# EUROPEAN PATENT APPLICATION

(11) **EP 2 926 831 A1**
(43) Date of publication of application: **07.10.2015**
(21) Application number: 14162790.1
(22) Date of filing: 31.03.2014
(51) Int. Cl.: A61K 39/12, C07K 14/005

(54) **CD8+ T-cell epitopes from polyomavirus capsid protein VP1 for vaccination**

(71) Applicant: Ruprecht-Karls-Universität Heidelberg, 69117 Heidelberg (DE)
(72) Inventor: Schmitt, Anita, Dr., 69120 Neckargemünd (DE); Schmitt, Michael, Dr., 69120 Neckargemünd (DE); Mani, Jiju, Emakulam. Kerala (IN); Malcherek, Georg, 69498 Schriesheim (DE)
(74) Representative: Grahn, Sibylla Maria

(57) **Abstract**

The present invention relates to vaccines comprising at least one compound which comprises at least one antigenic determinant or epitope of polyomavirus capsid protein (VP1), wherein the antigenic determinant or epitope is a CD8+ T cell epitope, preferably HLA-A2-restricted or HLA-B7-restricted. The present invention furthermore relates to nucleic acids encoding the compound(s) and the use of the vaccines and nucleic acids for the prevention and/or treatment of an infection or reactivation with polyoma virus(es), such as in case of immunosupression. The present invention furthermore relates to methods for the prevention and/or treatment of an infection or reactivation with polyoma viruses, comprising the step of administering a vaccine, nucleic acid or compound of the invention to a subject in need thereof.

## Description

The present invention relates to vaccines comprising at least one compound which comprises at least one antigenic determinant or epitope of polyomavirus capsid protein (VP1), wherein the antigenic determinant or epitope is a CD8+ T cell epitope, preferably HLA-A2-restricted or HLA-B7-restricted. The present invention furthermore relates to nucleic acids encoding the compound(s) and the use of the vaccines and nucleic acids for the prevention and/or treatment of an infection or reactivation with polyoma virus(es), such as in case of immunosuppression. The present invention furthermore relates to methods for the prevention and/or treatment of an infection or reactivation with polyoma viruses, comprising the step of administering a vaccine, nucleic acid or compound of the invention to a subject in need thereof.

### BACKGROUND OF THE INVENTION

The human immune system is finely balanced to maintain the equilibrium between protection from pathogens and tolerance to self-antigens. There are several factors that can disturb this equilibrium and hence lower the efficiency of immune responses. For example, a state of immunosuppression, that leads to the reactivation of various latent viral or bacterial pathogens, which are otherwise controlled by the efficient immune system. Immunosuppression can be caused by malnutrition, ageing or infection and can also be induced by immunosuppressive agents.

In general, immunosuppression is used in the clinical setting to prevent the host from rejecting a transplant or for the treatment of autoimmune diseases. Immunosuppression is induced mainly by drugs or radiation. Under immunosuppression, opportunistic infections occur due to the reactivation of latent viruses in the patients or from the donor organs. Viral reactivation is one of the major causes of mortality and morbidity in most of the transplanted patients. The most common opportunistic viral pathogen which is reactivated in a transplantation setting is CMV. Other viral pathogens like EBV, adenovirus, herpes simplex virus (HSV), BKV, and JCV are also reported in a significant number of cases.

Besides induction through immunosuppressant agents and radiation, immunosuppression occurs in the state of malnutrition, ageing or caused by infections (HIV or other diseases). HIV related immunosuppression is characterized by a progressive depletion of CD4⁺ helper T cells. This state of immunosuppression is also a major cause for the reactivation of many latent viruses like CMV, EBV and JCV (Stoner et al., 1998; Bienaime et al., 2006).

Human polyomaviruses were first described in the 1950s, when they were identified as the causative agents of multiple tumors in rodents, hence the name polyoma ("poly" greek for many and the greek suffix "-oma" for tumors) was coined. Simian virus-40 (SV-40) was the first primate polyomavirus identified in African green monkey kidney cells. JC and BK polyomaviruses were later described in 1970s. JCV was isolated from brain tissue of a patient with the initials "JC" who suffered from PML (Padgett et al., 1971), and BKV grew in cell cultures after inoculation with the urine from a renal transplant recipient with the initials "BK" (Sweet and Hilleman 1960). Eight additional human polyomaviruses have been identified since 2007.

The asymptomatic primary contact with polyomaviruses occurs during childhood or early adulthood resulting in a lifelong persistence in immunocompetent individuals, leading to more than 80% seroprevalence in the adult population worldwide. Initial infections of host cells are facilitated by the interactions of BKV and JCV with negatively charged sialic acid containing receptors. In addition to sialic acid as receptor, the JC virus also uses the serotonin receptor (5HT_{2A}R) to infect glial cells. After primary infection, human polyomaviruses persist in the urinary tract, B lymphocytes and the brain (Chesters et al., 1983).

Unlike BKV, the other member of same genus, JCV is never involved in diseases of the urogenital tract or the lungs. The most prominent site of JCV-associated complications in the immunocompromised host is the central nervous system (CNS). Once JCV has entered the CNS, the virus replicates vigorously and leads to progressive multifocal leucoencephalopathy (PML), a lethal disease. PML is a demyelinating disorder characterized by pinhead-sized demyelinating lesions in the brain. It occurs in 3-5% of HIV-infected patients, in patients suffering from autoimmune disorders, in cancer patients involving the lymphoid system (lymphoma), patients on long-term immunosuppressive therapy and few elderly patients with involution of the thymus. Previously, PML was considered as a rare opportunistic infection, but after the discovery of HIV, 80% of PML cases were contributed by HIV patients. The incidences of PML in non-HIV-infected patients are also increasing for hematologic malignancies (13%), organ transplant recipients (5%), and patients with autoimmune disease treated with immunomodulators (3%) (Gheuens et al., 2010). In PML disease, tissue demyelination occurs due to the lytic activity of JCV in oligodendrocytes, which leads to the impairment of brain function and eventually to death. However, highly active antiretroviral therapy (HAART) in HIV patients has significantly improved the survival rate of PML patients (De Luca et al., 2008). Nevertheless, the mortality associated with PML still approaches >50% within the first year.

The adverse effects of immunomodulatory therapy and immunosuppression are the major reason for immunosuppression leading to JCV-mediated fatal PML cases in patients. In immunosuppressed patients, immunomodulatory drugs like fludarabine, methotrexate, cyclophosphamide and monoclonal antibodies like natalizumab (in multiple sclerosis and Crohn's disease), rituximab (in MS, non-HL, RA, SLE, MG diseases) etc. are responsible for inhibiting the biological function of immune cells. As anti-polyomavirus drugs with clinical efficacy are simply not available, the treatment solely relies on improving virus-specific immunity.

So far, therapeutic approaches for PML have shown less or almost no significant clinical impact (Tavazzi et al., 2012). This could be due to the blood brain barrier which makes the CNS less accessible to chemical drugs. Nevertheless, a few clinical studies showed an improvement of clinical neurological symptoms and findings in the MRI after administration of risperidone, mitrazapine and recombinant IL-2 (Focosi et al., 2007; Cettomai and McArthur, 2009; Kuschner and Scott, 2005). One drug, cidofovir showed clinical impact in BKV-induced HC patients, however no significant clinical effect was reported on the survival of PML patients (De Luca et al., 2008). In contrast to that, an immunotherapeutic approach showed some promise: Here a patient with the clinical status of JCV-associated PML greatly improved and showed a complete clearance of JCV-DNA in the cerebrospinal fluid after the adoptive transfer of JCV-specific *in vitro* activated CTLs (Balduzzi et al., 2011). In summary, there are no clear guidelines for the drug treatment of JCV-related infection or reactivation and successful therapy depends greatly on the overall improvement of immune system. For example, in patients with PML, HAART improves the overall immune system hence it is the only therapy which seems to have an effective clinical outcomes.

US 6,238,859 discloses virus-like particles composed of the VP1 protein of the JC virus, in particular non-infectious virus-like particles comprising several molecules of the virus protein VP1 of the JC virus as the only JCV component in the virus like particle.

US 7,803,761 and US 8,158,576 describe viral capsid proteins, as a medicament for the treatment of disorders associated with inactivation of cellular proteins involved with quality control processes, particularly, chaperones, or the treatment of ARF (acute renal failure). More particularly, these patents relate to the medical use of the viral capsid proteins VP1, VP2 and VP3, in particular SV40 VP1 or a variant of SV40 VP1.

US 2009/0062237 relates to methods and materials for evaluating immune competence in a subject to BK virus (BKV). This application describes a multitude of peptides derived from BKV.

There is a need in the art for providing improved means and methods for the prevention and/or treatment of polyoma virus infections and/or reactivations in patients.

### SUMMARY OF THE INVENTION

According to the present invention this object is solved by providing a vaccine comprising at least one compound, said at least one compound comprising at least one antigenic determinant or epitope of polyomavirus capsid protein (VP1), wherein said at least one VP1 antigenic determinant or epitope is selected from

| | |
|---|---|
| IGVTSLMNV | SEQ ID NO. 1, |
| IGITSMLNL | SEQ ID NO. 2, |
| IGVTSLMNVHSNGQA | SEQ ID NO. 3, |
| IGITSMLNLHAGSQK | SEQ ID NO. 4, |
| KTEVIGVTSLMNVHS | SEQ ID NO. 5, |
| KTEVIGITSMLNLHA | SEQ ID NO. 6, |
| KRRVKNPYPISFLLT | SEQ ID NO. 7, |
| KRSVKNPYPISFLLS | SEQ ID NO. 8, |
| KNPYPISFLLTDLIN | SEQ ID NO. 9, |
| KNPYPISFLLSDLIN | SEQ ID NO. 10, |

| | |
|---|---|
| VARIPLPNL | SEQ ID NO. 11, |
| TARIPLPNL | SEQ ID NO. 12, and |
| KNPYPISFL | SEQ ID NO. 13. |

According to the present invention this object is solved by providing a nucleic acid molecule coding for at least one peptide as defined herein or a plasmid comprising at least one such nucleic acid molecule.

According to the present invention this object is solved by providing the nucleic acid according to the present invention or the vaccine according to the present invention for use in medicine.

According to the present invention this object is solved by providing the nucleic acid according to the present invention for use as a vaccine.

According to the present invention this object is solved by providing the vaccine according to the present invention or the nucleic acid according to the present invention for use for the prevention and/or treatment of an infection or reactivation with polyoma virus(es).

According to the present invention this object is solved by providing a compound comprising at least one antigenic determinant or epitope of polyomavirus capsid protein (VP1), wherein said at least one VP1 antigenic determinant or epitope is selected from

| | |
|---|---|
| IGVTSLMNV | SEQ ID NO. 1, |
| IGITSMLNL | SEQ ID NO. 2, |
| IGVTSLMNVHSNGQA | SEQ ID NO. 3, |
| IGITSMLNLHAGSQK | SEQ ID NO. 4, |
| KTEVIGVTSLMNVHS | SEQ ID NO. 5, |
| KTEVIGITSMLNLHA | SEQ ID NO. 6, |
| KRRVKNPYPISFLLT | SEQ ID NO. 7, |
| KRSVKNPYPISFLLS | SEQ ID NO. 8, |
| KNPYPISFLLTDLIN | SEQ ID NO. 9, |
| KNPYPISFLLSDLIN | SEQ ID NO. 10, |
| VARIPLPNL | SEQ ID NO. 11, |
| TARIPLPNL | SEQ ID NO. 12, and |

| | |
|---|---|
| KNPYPISFL | SEQ ID NO. 13, |

for use for the prevention and/or treatment of an infection or reactivation with polyoma virus(es).

According to the present invention this object is solved by a method for the prevention and/or treatment of an infection or reactivation with polyoma viruses, comprising the step of administering a vaccine according to the present invention, a nucleic acid according to the present invention or a compound according to the present invention to a subject in need thereof.

According to the present invention this object is solved by providing a compound comprising at least one antigenic determinant or epitope of polyomavirus capsid protein (VP1), wherein said at least one VP1 antigenic determinant or epitope is selected from

| | |
|---|---|
| IGVTSLMNV | SEQ ID NO. 1, |
| IGITSMLNL | SEQ ID NO. 2, |
| IGVTSLMNVHSNGQA | SEQ ID NO. 3, |
| IGITSMLNLHAGSQK | SEQ ID NO. 4, |
| KTEVIGVTSLMNVHS | SEQ ID NO. 5, |
| KTEVIGITSMLNLHA | SEQ ID NO. 6, |
| KRRVKNPYPISFLLT | SEQ ID NO. 7, |
| KRSVKNPYPISFLLS | SEQ ID NO. 8, |
| KNPYPISFLLTDLIN | SEQ ID NO. 9, |
| KNPYPISFLLSDLIN | SEQ ID NO. 10, |
| VARIPLPNL | SEQ ID NO. 11, and |
| TARIPLPNL | SEQ ID NO. 12. |

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art. For the purpose of the present invention, all references cited herein are incorporated by reference in their entireties.

Concentrations, amounts, and other numerical data may be expressed or presented herein in a range format. It is to be understood that such a range format is used merely for convenience and brevity and thus should be interpreted flexibly to include not only the numerical values explicitly recited as the limits of the range, but also to include all the individual numerical values or sub-ranges encompassed within that range as if each numerical value and sub-range is explicitly recited. As an illustration, a numerical range of "9 to 100 amino acids" should be interpreted to include not only the explicitly recited values of 9 to 100, but also include individual values and sub-ranges within the indicated range. Thus, included in this numerical range are individual values such as 9, 10, 11, 12, 13, 14, 15, 16, 17, 18, 19, 20,... 98, 99, 100 and sub-ranges such as from 9 to 50, from 9 to 25, from 10 to 25, from 10 to 20 and from 15 to 25, etc. This same principle applies to ranges reciting only one numerical value. Furthermore, such an interpretation should apply regardless of the breadth of the range or the characteristics being described.

### Compounds of CD8+ TCE peptides from VP1 as vaccines

As described above, the present invention provides a vaccine comprising at least one compound.

Said at least one compound of the invention comprises at least one antigenic determinant or epitope of polyomavirus capsid protein (VP1),
wherein said at least one VP1 antigenic determinant or epitope is selected from

| | | |
|---|---|---|
| p113 | IGVTSLMNV | SEQ ID NO. 1, |
| | IGITSMLNL | SEQ ID NO. 2, |
| OP29 | IGVTSLMNVHSNGQA | SEQ ID NO. 3, |
| | IGITSMLNLHAGSQK | SEQ ID NO. 4, |
| OP28 | KTEVIGVTSLMNVHS | SEQ ID NO. 5, |
| | KTEVIGITSMLNLHA | SEQ ID NO. 6, |
| OP72 | KRRVKNPYPISFLLT | SEQ ID NO. 7, |
| | KRSVKNPYPISFLLS | SEQ ID NO. 8, |
| OP73 | KNPYPISFLLTDLIN | SEQ ID NO. 9, |
| | KNPYPISFLLSDLIN | SEQ ID NO. 10, |

| | | |
|---|---|---|
| p83 | VARIPLPNL | SEQ ID NO. 11, |
| | TARIPLPNL | SEQ ID NO. 12, and |
| p289 | KNPYPISFL | SEQ ID NO. 13. |

The vaccines of the invention are preferably peptide-based vaccines.

Preferably, the antigenic determinant or epitope is a CD8⁺ T cell epitope,
preferably HLA-A2-restricted or HLA-B7-restricted.

A "T cell epitope" (TCE) is a peptide sequence which is derived from an antigen, binds to HLA molecule and can elicit an immunogenic T cell response. A CD8⁺ TCE peptide is also naturally processed inside the antigen presenting cells (APCs).

Epitope mapping is a process of identifying T cell determinants or TCE-specific to particular HLA restricted peptides from a relevant protein of pathogen. It is a crucial step in the development of peptide or T cell-based immunotherapies.

In a preferred embodiment, the amino acid sequence of the antigenic determinant or epitope has a length of at least 9 amino acids,
wherein the amino acid sequence of the antigenic determinant or epitope has a length of 9 to 100 amino acids, preferably 9 to 50 amino acids, more preferably 9 to 25 amino acids.

In a preferred embodiment, the the antigenic determinant or epitope has a length of 9 amino acids or 15 amino acids. The polyomavirus capsid protein (VP1) of JC virus (JCV):
GenBank Accession No.: AAA82101.1, 354 amino acids
UniProt Accession No.: P03089

10 20 30 40 50 60
MAPTKRKGER KDPVQVPKLL IRGGVEVLEV KTGVDSITEV ECFLTPEMGD PDEHLRGFSK

70 80 90 100 110 120
SISISDTFES DSPNRDMLPC YSVARIPLPN LNEDLTCGNI LMWEAVTLKT EVIGVTSLMN

130 140 150 160 170 180
VHSNGQATHD NGAGKPVQGT SFHFFSVGGE ALELQGVLFN YRTKYPDGTI FPKNATVQSQ

190 200 210 220 230 240

VMNTEHKAYL DKNKAYPVEC WVPDPTRNEN TRYFGTLTGG ENVPPVLHIT NTATTVLLDE

250 260 270 280 290 300
FGVGPLCKGD NLYLSAVDVC GMFTNRSGSQ QWRGLSRYFK VQLRKRRVKN PYPISFLLTD

310 320 330 340 350
LINRRTPRVD GQPMYGMDAQ VEEVRVFEGT EELPGDPDMM RYVDKYGQLQ TKML

The amino acid sequence is also shown in SEQ ID NO. 14.

The polyomavirus capsid protein (VP1) of BK virus (BKV)
Accession No.: P14996, 362 amino acids

10 20 30 40 50 60
MAPTKRKGEC PGAAPKKPKE PVQVPKLLIK GGVEVLEVKT GVDAITEVEC FLNPEMGDPD

70 80 90 100 110 120
DNLRGYSQHL SAENAFESDS PDRKMLPCYS TARIPLPNLN EDLTCGNLLM WEAVTVKTEV

130 140 150 160 170 180
IGITSMLNLH AGSQKVHENG GGKPVQGSNF HFFAVGGDPL EMQGVLMNYR TKYPQGTITP

190 200 210 220 230 240
KNPTAQSQVM NTDHKAYLDK NNAYPVECWI PDPSRNENTR YFGTYTGGEN VPPVLHVTNT

250 260 270 280 290 300
ATTVLLDEQG VGPLCKADSL YVSAADICGL FTNSSGTQQW RGLARYFKIR LRKRSVKNPY

310 320 330 340 350 360
PISFLLSDLI NRRTQKVDGQ PMYGMESQVE EVRVFDGTEQ LPGDPDMIRY IDRQGQLQTK

MV

The amino acid sequence is also shown in SEQ ID NO. 15.

### Disclaimer:

A compound in a vaccine of the invention does not comprise a full-length viral capsid protein, such as VP1 or VP2, VP3 or others, such as VP1 of JCV or VP1 ofBKV.

In a preferred embodiment, said at least one compound of the vaccine is a peptide, preferably a peptide consisting of said at least one VP 1 antigenic determinant or epitope.

In one embodiment, the compound, such as a peptide, according to the present invention comprises at least two VP1 antigenic determinants or epitopes. In further embodiments, the compounds, such as peptides, of the present invention comprise 3, 4, 5 or more such antigenic determinants or epitopes.

In one embodiment, the vaccine comprises further component(s), such as
- tag(s),
   such as 6-His-tag, Flag-tag,
- linker,
   such as polylinkers to link one (poly)peptide to another (poly)peptide
- label(s),
   such as radioisotopes (like indium-111), enzymes, luminescent or fluorescent labels,
- N- and/or C-terminal modification(s),
   such as comprising acetylation and/or amidation of the *N*- and/or C-terminus,
- drug(s),
   such as ozogamicin, ricin A, anthracyclins,
- agent(s),
and/or
combinations thereof.

The skilled artisan will be able to select suitable further components.

Said further component(s) can be covalently attached to the compound or peptide of the vaccine.

In one embodiment, the amino acid sequence comprises modified amino acid(s), unnatural amino acid(s) or peptidomimetic(s).

In one embodiment, the vaccine of the present invention comprises at least two compounds (preferably two peptides) according to the invention.

In further embodiments, the vaccine(s) of the present invention comprise 3, 4, 5 or more of the compounds (such as peptide(s)) of the invention.

In a preferred embodiment, the vaccine further comprises
- carrier(s), such as a virus like particle (VLP),
   and/or
- adjuvant(s).

Examples for suitable adjuvants are
- montanide™,
- AS02 Adjuvant System,
   such as AS02-01,
   the AS02 Adjuvant System is the combination of an oil-in-water emulsion with MPL (3-de-O-acylated monophosphoryl lipid A) and QS-21.
- Adjuvant QS-21: is an immunological adjuvant derived from a natural source: the bark of the South American tree *Quillaja saponaria* Molina.
- Imiquimod: 1-isobutyl-1H-imidazo[4,5-c] quinolin-4-amine
- other Toll-like-receptor ligands,
- oligodinucleotides of classes A, B and C.

Since peptides are usually poor immunogens, the efficacy of peptide-based vaccine depends on the adequate presentation of the epitopes to the immune system.

The skilled artisan will be able to select suitable carrier(s) and/or adjuvant(s).

In one embodiment, the carrier is a virus particle or parts thereof, an envelope protein of a viral vector or of a virus particle, a nanocarrier or a liposomal compound enveloping the peptide(s) in a micellular structure.

In one embodiment, the carrier is a virus like particle (VLP).
"Virus-like particles" (VLPs) resemble viruses in size and shape, but are non-infectious because they do not contain any viral genetic material. Therefore, VLPs represent excellent antigens for various applications. The expression of viral structural proteins, such as Envelope or Capsid, can result in the self-assembly of virus like particles (VLPs). VLPs can be produced in a variety of expression systems including *Escherichia coli,* mammalian cell lines, insect cell lines, yeast, and plant cells.

The vaccines of the present invention can further comprise excipient(s), such as pharmaceutically acceptable excipient(s), which can be necessary for the different administration regimes (e.g. via injection, such as subcutaneously or intradermally).

Further suitable excipient(s) are known to the skilled artisan.

### Nucleic acids

As described above, the present invention provides a nucleic acid molecule coding for at least one compound of the vaccine according to the present invention (and as defined above), preferably coding for at least one peptide of the invention.

As described above, the present invention provides a plasmid comprising at least one such nucleic acid molecule.

The nucleic acid molecules can be isolated nucleic acid molecules, plasmids comprising them, expression constructs comprising them, such as expression systems for DNA vaccines.

Examples of nucleic acid(s) of the invention are DNA or RNA encoding the compounds/peptides of the invention.

The nucleic acid molecules of the invention, such as said above DNA or RNA encoding the compounds/peptides of the invention, are suitable for the transfection into antigen-presenting cells, such as DCs.

### Medical uses of the vaccines and nucleic acids

As described above, the present invention provides the vaccine of the present invention or the nucleic acid of the present invention for use in medicine.

As described above, the present invention provides the nucleic acid of the present invention for use as a vaccine.

As described above, the present invention provides the vaccine of the present invention or the nucleic acid of the present invention for use for the prevention and/or treatment of an infection or reactivation with polyoma virus(es).

Preferably, the polyoma virus is JC virus (JCV) or BK virus (BKV).

Polyomaviruses are small non-envelope particles encompassing the circular double-stranded deoxyribonucleic acid (DNA) genome. BKV and JCV genomes are approximately 5,300 bp and 5,130 bp in size, respectively (Ferenczy et al., 2012). All members of the polyomavirus family share common morphology and genome organization. The polyomavirus genome can be divided into three functional parts.
(i) The non-coding control region: contains viral promoters and the origin of replication.
(ii) The viral early gene region which encodes the large T-antigen (LT-antigen) and the small T-antigen (sT-antigen). It is involved in the replication of viral genome.
(iii) The viral late gene region which encodes the viral proteins VP1, VP2, VP3 as well as the agno-protein. It is involved in encoding structural proteins.

Among all the early and late gene expressing proteins, VP1 is the only protein exposed on the surface of the capsid and thus determines the receptor specificity.

Preferably, the use comprises
- vaccination,
- adoptive immunotherapy,
- T-cell receptor cloning and transfection,
- construction of chimeric antigen receptors.

Said T-cell receptor cloning and transfection comprises
- cloning of T cells recognizing specifically the compound(s) of the invention (such as peptides),
- molecular cloning of a T cell receptor (TCR) from said T cell clones, and
- transfection of such TCRs, preferably into autochthonous T lymphocytes of the subject in need thereof / the patient.

In one embodiment, instead of a TCR, also chimeric antigen receptors is constructed and cloned.

Preferably, the use is carried out in case of/during immunosuppression.

Such as
- in patients after transplantation,
   such as stem cell or organ transplantation,
- HIV infection,
- patients with solid tumors or hematological malignancies,
   such as multiple myeloma or chronic lymphcyctic leukemia,
- patients with
   rheumatologic disorders, preferably systemic lupus erythematosus (SLE)), lympho-proliferative disorders,
   during monoclonal antibody (mAb) therapy.

For example, JCV causes PML during immunosuppressive conditions like the following:
(1) Infections with HIV: The majority of the PML cases have been reported in HIV-1 patients. A very few cases are also reported in HIV-2 patients (Stoner et al., 1998; Bienaime et al., 2006). With the advent of HIV treatment, the so-called HAART there was a significant improvement in the survival time of PML patients in HIV settings. However, PML is still the second most common cause of death (14%) in HIV patients (Lewden et al., 2008).
(2) In the setting of hematologic and oncologic malignancies: JCV-induced PML is also associated with lympho-proliferative disorders (Garcia-Suarez et al., 2005), like in chronic lymphocytic lymphoma, Hodgkin disease, non-Hodgkin lymphoma and multiple myeloma.
(3) In the setting of stem cell and solid organ transplantations: JCV-reactivation occurs in transplantation setting due to the immunosuppressive conditions induced by intense immunosuppressive regimen (Shitrit et al., 2005; Kharfan-Dabaja et al., 2007).
(4) In patients with rheumatologic disorder: Systemic lupus erythematosus (SLE) is most commonly associated with JCV-induced PML. In other rheumatologic disorders like rheumatoid arthritis, dermatomyositis, polymyositis and scleroderma the incidence of PML is rather low.
(5) During monoclonal antibody (mAb) therapy: mAb treatment is practiced in many immunological diseases and few of these antibodies can suppress the immune system. For example, antibodies like, efalizumab, rituximab and natalizumab which are used in the setting of autoimmune diseases are associated with PML (Kleinschmidt-DeMasters and Tyler, 2005; Langer-Gould et al., 2005; Carson et al., 2009).
(6) Other diseases: Few conditions of no or minimal immunodeficiency like renal failure, psoriasis, dermatomyositis etc. are also reported to cause immunosuppression and make patients prone to PML (Gheuens et al., 2010).

Viral infection during immunosuppression is a major complication in transplantation, rheumatologic, and other immune-deficient condition (HIV).

"Immunotherapy" is one effective approach which is defined as the treatment of disease by manipulating the immune system of patients. Immunotherapeutic approaches have been evolved from infusion of non-antigen-specific T cells, a donor lymphocyte infusion (DLI) to the selection and isolation of antigen-specific T cells during the past few decades. During DLI, a large number of donor T cells are also transfused into the host. Although these mature T cells are responsible for the protection against infectious diseases, they are also largely responsible for the unintended graft versus host disease (GvHD). To overcome this problem one option is to reconstitute the immune system by the adoptive transfer of donor T cells. This should be performed after few weeks or months from transplantation, in order to reduce the risk of GvHD. Moreover, when adoptive transfer is performed using peptide epitope-specific T cells the risk of GvHD is almost nil.

In the case of JCV-associated infections, a single case study using JCV-specific T cells as adoptive T cell therapy has been reported (Balduzzi et al., 2011). Here, JCV-stimulated T cells were not single-epitope-specific rather, PBMCs were expanded using 15mer peptide pool of entire JCV-VP1 and LT-antigen proteins.

For an effective antigen-specific immunotherapeutic strategy, the knowledge of HLA-restriction and selection of a potent immunogenic peptide antigen is the most critical step.

As described above, the present invention further provides a compound comprising at least one antigenic determinant or epitope of polyomavirus capsid protein (VP1),
wherein said at least one VP1 antigenic determinant or epitope is selected from

| | | |
|---|---|---|
| p113 | IGVTSLMNV | SEQ ID NO. 1, |
| | IGITSMLNL | SEQ ID NO. 2, |
| OP29 | IGVTSLMNVHSNGQA | SEQ ID NO. 3, |
| | IGITSMLNLHAGSQK | SEQ ID NO. 4, |
| OP28 | KTEVIGVTSLMNVHS | SEQ ID NO. 5, |
| | KTEVIGITSMLNLHA | SEQ ID NO. 6, |
| OP72 | KRRVKNPYPISFLLT | SEQ ID NO. 7, |
| | KRSVKNPYPISFLLS | SEQ ID NO. 8, |
| OP73 | KNPYPISFLLTDLIN | SEQ ID NO. 9, |
| | KNPYPISFLLSDLIN | SEQ ID NO. 10, |
| p83 | VARIPLPNL | SEQ ID NO. 11, |
| | TARIPLPNL | SEQ ID NO. 12, and |
| p289 | KNPYPISFL | SEQ ID NO. 13, |

for use for the prevention and/or treatment of an infection or reactivation with polyoma virus(es).

The embodiments of said use are as described above.

The compound is preferably as defined above.

### Disclaimer:

A compound of the invention does not comprise a full-length viral capsid protein, such as VP1 or VP2, VP3 or others, such as VP1 of JCV or VP1 of BKV.

### Methods of preventing and/or treating polyoma virus infections/reactivations

As described above, the present invention provides a method for the prevention and/or treatment of an infection or reactivation with polyoma viruses.

Said method comprises the step of
administering a vaccine according to the present invention, a nucleic acid of the present invention or a compound as defined in the present invention to a subject in need thereof.

Said administration to a subject in need thereof can be carried out
(1) via injection,
   such as injection subcutaneously (s.c.) or intradermally (i.d.),
(2) pulsing of compound(s) of the invention (e.g. peptides) on dendritic cells (DCs) which are thereafter administered,
   such as intravenously (i.v.), s.c. or i.d.,
(3) stimulation of T cells with compound(s) of the invention (e.g. peptide) *ex vivo* and transfusion of such stimulated and potentially selected T cells,
   such as via i.v. transfusion,
(4) transfection of nucleic acid(s) of the invention (e.g. DNA or RNA encoding the peptide(s)) into antigen-presenting cells, such as DCs,
(5) cloning of T cells recognizing specifically the compound(s) of the invention (e.g. peptides), expansion of these T clones *ex vivo* followed by injection,
   such as i.v. injection,
(6) molecular cloning of a T cell receptor (TCR) from the T cell clones as described above in (5) and transfection of such TCRs into autochthonous T lymphocytes of the subject in need thereof / the patient.

Preferably, the subject in need thereof is an immune-suppressive patient.

Such as
- a patient after transplantation (such as stem cell or organ transplantation,
- a HIV-infected patient,
- a patient with solid tumors or hematological malignancies (such as multiple myeloma or chronic lymphcyctic leukemia)
   or
- a patient with rheumatologic disorders (such as systemic lupus erythematosus (SLE)), lympho-proliferative disorders, during monoclonal antibody (mAb) therapy.
   as described above.

Preferably, the polyoma virus is JC virus (JCV) or BK virus (BKV), as described above.

In one embodiment, the method comprises
- vaccination,
- adoptive immunotherapy,
   also
- T-cell receptor cloning and transfection,
- construction of chimeric antigen receptors.

### Compounds of CD8+ TCE peptides from polyomavirus capsid protein VP1

As described above, the present invention provides a compound comprising at least one antigenic determinant or epitope of polyomavirus capsid protein (VLP1),
wherein said at least one VP1 antigenic determinant or epitope is selected from

| | | |
|---|---|---|
| p113 | IGVTSLMNV | SEQ ID NO. 1, |
| | IGITSMLNL | SEQ ID NO. 2, |
| OP29 | IGVTSLMNVHSNGQA | SEQ ID NO. 3, |
| | IGITSMLNLHAGSQK | SEQ ID NO. 4, |
| OP28 | KTEVIGVTSLMNVHS | SEQ ID NO. 5, |
| | KTEVIGITSMLNLHA | SEQ ID NO. 6, |
| OP72 | KRRVKNPYPISFLLT | SEQ ID NO. 7, |
| | KRSVKNPYPISFLLS | SEQ ID NO. 8, |
| OP73 | KNPYPISFLLTDLIN | SEQ ID NO. 9, |
| | KNPYPISFLLSDLIN | SEQ ID NO. 10, |
| p83 | VARIPLPNL | SEQ ID NO. 11, and |
| | TARIPLPNL | SEQ ID NO. 12. |

As described above, preferably, the antigenic determinant or epitope is a CD8⁺ T cell epitope, preferably HLA-A2-restricted or HLA-B7-restricted.

As described above, in a preferred embodiment, the amino acid sequence of the antigenic determinant or epitope has a length of at least 9 amino acids,
wherein the amino acid sequence of the antigenic determinant or epitope has a length of 9 to 100 amino acids, preferably 9 to 50 amino acids, more preferably 9 to 25 amino acids.

As described above, in a preferred embodiment, the the antigenic determinant or epitope has a length of 9 amino acids or 15 amino acids.

### Disclaimer:

A compound of the invention does not comprise a full-length viral capsid protein, such as VP1 or VP2, VP3 or others, such as VP1 of JCV or VP1 ofBKV.

As described above, in a preferred embodiment, the compound of the invention is a peptide, preferably a peptide consisting of said at least one VP1 antigenic determinant or epitope.

As described above, in one embodiment, the compound, such as a peptide, according to the present invention comprises at least two VP1 antigenic determinants or epitopes. In further embodiments, the compounds, such as peptides, of the present invention comprise 3, 4, 5 or more such antigenic determinants or epitopes.

As described above, in one embodiment, the compound/peptide comprises further component(s), such as
- tag(s),
   such as 6-His-tag, Flag-tag,
- linker,
   such as polylinkers to link one (poly)peptide to another (poly)peptide
- label(s),
   such as radioisotopes (like indium-111), enzymes, luminescent or fluorescent labels,
- N- and/or C-terminal modification(s),
   such as comprising acetylation and/or amidation of the *N*- and/or C-terminus,
- drug(s),
   such as ozogamicin, ricin A, anthracyclins,
- agent(s),
and/or
combinations thereof.

The skilled artisan will be able to select suitable further components.

As described above, in one embodiment, the amino acid sequence comprises modified amino acid(s), unnatural amino acid(s) or peptidomimetic(s).

### Preferred embodiments

Reactivation of polyomaviruses JCV and BKV in immunocompromised patients after transplantation results in severe complications related to the central nervous system (CNS) and the urogenital system, i.e. progressive multifocal leukoencephalopathy (PML) and hemorrhagic cystitis (HC). Anti-viral drug treatment in this situation is not effective and therefore cure is largely dependent on the restoration of the immune system by tapering out of immunosuppressive drugs. However, restoration of immune system post transplantation might lead to graft versus host disease (GvHD). Adoptive transfer of virus-specific T cells constitute an alternative would avoid the risk of GvHD due to its specific reactivity to virus-infected cells.

To this end, two previously known immunodominant human leukocyte antigen (HLA)-A2 restricted epitopes (p36 and p100) derived from VP1 protein were analyzed. We observed that the precursor T cell frequency to these two HLA-A2 restricted peptides was very low when compared to T cells specific for other viruses (e.g. CMV, influenza virus etc.). To efficiently measure polyomavirus-specific T cells *in vitro,* optimization towards increased sensitivity of the expansion protocol and the read-out system was performed. Further characterization showed that p100-specific T cells were T cells of high avidity. Moreover, in *the in vitro* culture, virus-specific T cells were successfully maintained for a longer period of time (up to 5 weeks) with no loss of peptide-specificity and functional reactivity. Additionally, we demonstrated that JCV-VP1-peptide-specific T cells were cross-reactive to the BKV-VP1-p108-peptide when measured by multi-parametric flow cytometry and functional assays like ELISPOT and cytotoxicity assays.

In a comprehensive approach to widen our knowledge for new CD8⁺ TCEs restricted to other HLA-types, we used an array of overlapping peptides spanning the whole JCV-VP1 protein. Peptides were used in a form of matrix sub-pools and/or as individual peptide stimulation. In addition to two known HLA-A2 restricted peptide epitopes, several novel T cell specificities were identified in healthy donors (HDs) and in a patient. Using VP1-derived virus like articles (VLP) for initial stimulation, it was confirmed that all responding peptides were naturally processed. Additionally, a restriction analysis was performed for HLA-A2 and HLA-B7 alleles using HLA class I transfected lines of K562 and T2 cells as target cells in the immunological assays.

Taken together, we observed JCV-VP1-specific T cells to two HLA-A2 restricted immunodominant peptides of high avidity with sustained peptide-specificity and high functional reactivity in long-term cultures. Furthermore, we identified novel, naturally processed JCV-VP1-derived CD8⁺ T cell epitopes (TCEs) restricted to HLA-A2 and HLA-B7. This new set of data will broaden the armamentarium of therapeutically useful peptides and contribute to the development of T cell immunotherapies against polyomavirus-related disease. Our long term goal includes enrichment and isolation of polyomavirus-specific T cell specificities for use as adoptive T cell transfer in the clinical setting. Cross-reactive T cells have the additional benefit that an appropriate immunotherapy can target both polyomaviruses simultaneously.

For JCV and BKV-associated diseases in immunocompromised patients, adoptive T cell transfer constitutes an excellent option. To date two immunodominant CD8⁺ T cell epitopes have been identified in HLA-A2 individuals. To provide adoptive T cell transfer to patients across all haplotypes, there is a fervent need to identify and characterize more T cell epitopes. In the clinical setting, this extended knowledge would open new avenues for the efficient development of a comprehensive T cell immunotherapy against JCV and BKV infection.
The specific aim of this study was as follows:
(1) Characterization of immunodominant HLA-A2 restricted JCV-VP1-p100-peptide-specific T cells.
(2) Characterization of the cross-reactivity between JCV and BKV-peptide-specific T cells.
(3) Identification of novel, naturally processed HLA-A2 and HLA-B7 restricted CD8⁺ T cell epitopes in healthy donors (HDs) and patients.

### RESULTS

- *Assessment of novel JCV-VP1* CD8⁺ *T cell epitopes (TCEs)*
- *Novel T cell epitopes (TCEs) in HDs*

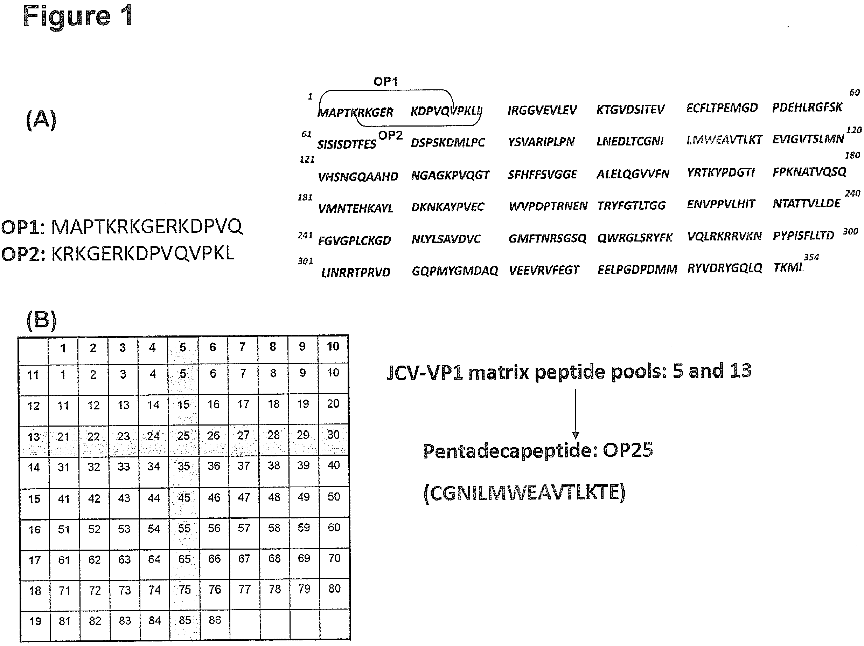

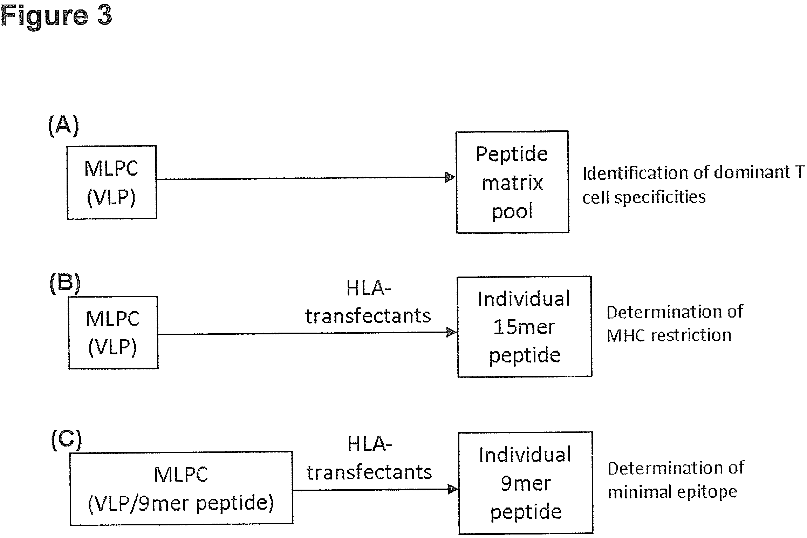

In order to map T cell antigens, a comprehensive approach using 19 JCV-VP1-peptide sub-pools was employed by the inventors in a matrix setup (see Figure 1). All healthy donors (HDs) were first HLA-typed by flow cytometry and SSP-PCR methods. Donors, who were positive for HLA-A2 and HLA-B7 were selected and analyzed for T cell reactivity to the JCV-VP1-peptide pool. Samples from HDs showing positive immune responses were then used for further identification of potential TCEs. A systematic scheme for identification of TCEs to JCV-VP1 is displayed in Figure 3. The first step was to identify dominant T cell specificities using the peptide matrix pool approach (Figure 3 A). Here, the stimulation (at day 0) was initiated by JCV-VLP. This was a very important step, as it confirmed that the stimulating antigenic peptides were not artificial but naturally processed. After identification of a particular T cell specificity, restriction analysis was performed using HLA-transfectants loaded with individual 15mer peptides (Figure 3 B). For this purpose, T2 cells and K562 cells transfected with HLA-A2 or HLA-B7 alleles were used as APCs. To further characterize the minimal epitope, 9mer peptides were predicted by the use of the SYFPEITHI software (http://www.syfpeithi.de/), synthesized and then tested individually (Figure 3 C).

By IFN-γ ELISPOT assays, strength of T cell responses was estimated using stimulation index (S.I) which was calculated for each positive signal after dividing the number of dots in the experimental wells in the 96-well plate with the negative control wells. Based on this index, T cell responses were classified as weak (between 2 and 3), moderate (between 3 and 4) or strong (≥4).

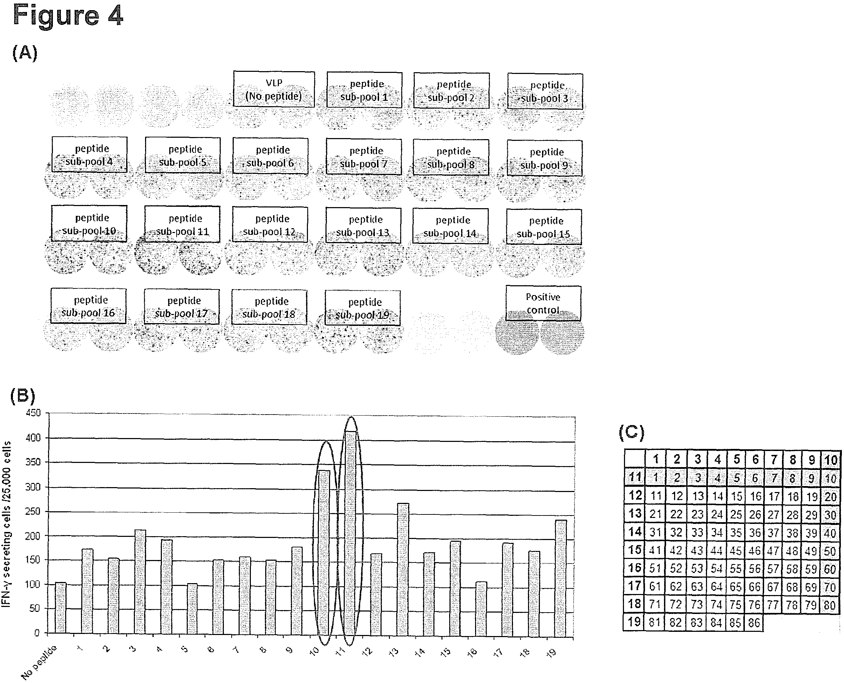

The first step, i.e. the identification of T cell specificities using the peptide matrix pool is a very crucial one. A representative experiment employing peptide matrix pool stimulation is depicted in Figure 4. In this representative example, JCV-VLP-stimulated CD8⁺ T cells were restimulated with 19 peptide matrix sub-pools in an ELISPOT plate with each restimulation in duplicate. Complete medium and SEB stimulation served as negative and positive controls, respectively. Two positive responding peptide sub-pools (sub-pools 10 and 11) were identified on the basis of number of IFN-γ secreting cells (ellipses) (Figure 4 A, B). In a further step, the common peptide present in both peptide sub-pools was selected with the help of matrix grid (Figure 4 C). This 15mer peptide (in this experiment: OP10) was used in subsequent experiments to analyze the T cell reactivity.

### - Retrieval of previously known JCV-VP1 T cell epitopes

Using the matrix approach, we were able to confirm two previously known HLA-A2 restricted JCV-VP1 epitopes p36 and p100. Using the two 15mer peptides OP9 and OP25 containing these epitopes, we found T cell reactivity in more than 4 out of 12 donors (Table 1). Additionally, p36 and p100-peptides were used in all 22 HLA-A2 positive donors tested and we found that 31% donors were positive for p36 and 41% donors were positive for p100-peptides (Table 3).

### - Identification of novel T cell specificities

In addition to reactivity to p36 and p100-peptides, we found several other specificities in HLA-A2 positive donors (Table 1). Some of the T cell responses to a particular antigenic peptide were shared by more than three HDs (≥25% HDs), for e.g. the peptides OP9, OP25, OP28, OP29, OP72 and OP78. In four different donors, stimulation by the two 15mers OP72 and OP78 resulted in positive signals. Especially donor#4318 showed a strong T cell response to the OP72 peptide in three independent experiments. The peptide OP29 was another 15mer peptide with strong signals in 33% of the donors we tested. Using the SYFPEITHI software, the 9mer peptide named p113 was predicted. The peptide was synthesized and used in experiments with twelve HLA-A2 positive donors. We observed that 3 out 12 donors tested were positive for T cell responses to peptide p113 including the HD#0132 who showed an exceptionally strong T cell response.

**Table 1 Antigenic peptides tested in HLA-A2 positive healthy donors (HDs).**

| **JCV-VP1-peptides** | **ID** | ***In silico* predicted T cell epitope** | **Experimental T cell reactivity** |
|---|---|---|---|
| GVDSITEVECFLTPE (OP9) [SEQ ID NO. 16] | 0196 | S **I** T E V E C F **L** [SEQ ID NO. 28] | Weak |
| | 6551 | | Moderate |
| | 2004 | | Moderate |
| | 4195 | | Moderate |
| | 4318 | | Moderate |
| MLPCYSVARIPLPNL (OP20) | 0196 | V **A** R I P L P N **L** [SEQ ID NO. 29] | Moderate |
| CGNILMWEAVTLKTE (OP25) [SEQ ID NO. 18] | 0196 | I **L** M W E A V T **L** [SEQ ID NO. 30] | Moderate |
| | 4895 | | Moderate |
| | 0132 | | Moderate |
| | 2004 | | Moderate |
| KTEVIGVTSLMNVHS (OP28) (SEQ ID NO. 3) | 4895 | T **E** V I G V T S **L** [SEQ ID NO. 31] | Moderate |
| | 2004 | | Weak |
| | 5728 | | Moderate |
| IGVTSLMNVHSNGQA (OP29) (SEQ ID NO. 2) | 0196 | I **G** V T S L M N **V** (SEQ ID NO. 1) | Strong |
| | 4895 | | Moderate |
| | 2004 | | Weak |
| | 5728 | | Moderate |
| PVECWVPDPTRNENT (OP50) | 0196 | W **V** P D P T R N **E** [SEQ ID NO. 32] | Moderate |
| PTRNENTRYFGTLTG (OP52) | 0196 | E **N** T R Y F G T **L** [SEQ ID NO. 33] | Moderate |
| VDVCGMFTNRSGSQQ (OP65) | 0196 | G **M** F T N R S G **S** [SEQ ID NO. 34] | Weak |
| KRRVKNPYPISFLLT (OP72) (SEQ ID NO. 5) | 4318 | K **N** P Y P I S F **L** (SEQ ID NO. 4) | Strong |
| | 2004 | | Moderate |
| | 0065 | | Weak |
| | 5728 | | Moderate |
| KNPYPISFLLTDLIN (OP73) | 4318 | P **I** S F L L T D **L** [SEQ ID NO. 35] | Moderate |
| VDGQPMYGMDAQVEE (OP78) [SEQ ID NO. 23] | 0132 | P **M** Y G M D A Q **V** [SEQ ID NO. 36] | Moderate |
| | 5728 | | Moderate |
| | 0014 | | Weak |
| | 0196 | | Weak |
| PMYGMDAQVEEVRVF (OP79) | 0132 | G **M** D A Q V E E **V** [SEQ ID NO. 37] | Moderate |
| GTEELPGDPDMMRYV (OP83) | 0196 | E **L** P G D P D M **M** [SEQ ID NO. 38] | Moderate |
| IGVTSLMNV (p113) (SEQ ID NO. 1) | 4895 | I **G** V T S L M N **V** [SEQ ID NO. 39] | Moderate |
| | 132 | | Strong |
| | 0196 | | Weak |
| ILMEWEAVTL (p100) (already described) [SEQ ID NO. 26] | 0992 | I **L** M W E A V T **L** | Strong |
| | 4090 | | Strong |
| | 0132 | | Moderate |
| | 0207 | | Strong |
| | 2564 | | Strong |
| | 2004 | | Moderate |
| | 4895 | | Strong |
| | 4195 | | Moderate |
| | 4318 | | Moderate |
| SITEVECFL (p36) (already described) [SEQ ID NO. 27] | 0196 | S **I** T E V E C F **L** | Strong |
| | 4129 | | Weak |
| | 2564 | | Moderate |
| | 2004 | | Weak |
| | 4195 | | Strong |
| | 4318 | | Moderate |
| | 4895 | | Weak |

In HLA-B7 positive donors, we observed moderately positive T cell responses to 15mer peptides at a frequency of one out of ten HDs (Table 2).

**Table 2 Antigenic peptides tested in ten HLA-B7 positive healthy donors (HDs).**

| **JCV-VP1-peptides** | **ID** | ***In silico* predicted T cell epitope** | **Experimental T cell reactivity** |
|---|---|---|---|
| TPEMGDPDEHLRGFS (OP12) | 1608 | D **P** D E H L R G **F** | Weak |
| GDPDEHLRGFSKSIS (OP13) | 4106 | D **P** D E H L R G **F** | Strong |
| ESDSPNRDMLPCYSV (OP18) | 4169 | S **D** S P N R D M **L** | Moderate |
| PNLNEDLTCGNILMW (OP23) | 4106 | E **D** L T C G N **I L** | Moderate |
| | 4196 | | Weak |
| HDNGAGKPVQGTSFH (OP33) | 4106 | K P **V** Q **G** T S F **H** | Weak |
| VLFNYRTKYPDGTIF (OP40) | 4106 | R **T** K Y P D G T **I** | Moderate |
| ENTRYFGTLTGGENV (OP53) | 4106 | E **N** T R Y F G T **L** | Weak |
| | 4196 | | Moderate |
| PVLHITNTATTVLLD (OP57) | 4106 | **I T** N T A T T V **L** | Moderate |
| PLCKGDNLYLSAVDV (OP62) | 4169 | L **C** K G D N L Y **L** | Moderate |
| KRRVKNPYPISFLLT (OP72) | 4169 | N **P** Y P I S F L **L** | Moderate |
| LINRRTPRVDGQPMY (OP76) | 4196 | T **P** R V D G Q **P M** | Moderate |
| RVFEGTEELPGDPDM (OP82) | 1608 | R **V** F E G T E E **L** | Moderate |
| GPLCKGDNL (p244) | 4278 | G **P** L C K G D N **L** | Strong |
| NPYPISFLL (p290) | 1608 | N **P** Y P I S F L **L** | Moderate |
| | 4106 | | Weak |
| VARIPLPNL (p83) | 1608 | V **A** R I P **L** P N **L** | Moderate |
| (SEQ ID NO. 7) | 4106 | | Weak |
| | 4195 | | Moderate |

In summary, we observed that among all HLA-B7 restricted T cell specificities given in Table 2, two out of ten donors showed T cell responses to the peptides OP23, OP53 and p290, whereas three out of ten donors tested were positive for the 9mer peptide p83. The p83-peptide was also shown to be immunogenic in one patient sample (discussed below).

Based on the frequency and strength of T cell responses in HDs, some of the candidate TCEs including p36 and p100 are summarized in Table 3. It was observed that the frequency of positive donors for all candidate TCEs were in the range of 25% to 41% with the top score for p100-peptide. To estimate the relative strength of T cell stimulation by each antigen peptide in a responding donor, we calculated the magnitude (index) of T cell responses. For example, a strong, moderate and weak T cell response to a particular antigenic peptide in a single donor was given a magnitude (index) of 1, 0.66 and 0.33 respectively. Based on this calculation, it was observed that in a positively responding HD, T cell responses to p100-peptide were very high while T cell responses to OP78 peptide were of lowest magnitude.

**Table 3. Donor frequencies specific to potential TCEs.**

| **JCV-VP1-peptides** | **Total donors tested** | **Positive donors** | **Magnitude (index) of T cell response (maximum 1)** |
|---|---|---|---|
| p83 (SEQ ID NO. 7) | 10 | 3 (30%) | 0.55 |
| OP29 (SEQ ID NO. 2) | 12 | 4 (33%) | 0.66 |
| OP72 (SEQ ID NO. 5) | 12 | 4 (33%) | 0.58 |
| OP78 | 12 | 4 (33%) | 0.50 |
| p113 (SEQ ID NO. 1) | 12 | 3 (25%) | 0.66 |
| p100 | 22 | 9 (41%) | 0.86 |
| p36 | 22 | 7 (31 %) | 0.59 |

In summary, we could observe several novel candidate HLA class I-TCEs in HLA-A2 and HLA-B7 positive HDs using our approach. Additionally, previously described HLA-A2 restricted immunodominant peptides were confirmed. Using JCV-VLP as primary stimulus, we demonstrated that all newly defined TCEs were naturally processed.

### - Novel T cell epitopes (TCEs) in a patient

In addition to HDs, we had the opportunity to analyze a patient for polyoma-reactive CD8⁺ T cells. The HLA class I and class II haplotype is HLA-A3, HLA-A29; HLA-B7, HLA-B44; HLA-CW*07, HLA-CW*16; HLA-DRB1*0101, HLA-DRB1*0701; HLA-DQB1*05; HLA-DQB1*02; HLA-DQA1*0101; HLA-DQA1*0201.

This patient had a history of a severe HC. Due to his previous exposure to BKV, we decided to analyze the T cell response to BKV-VLP. In parallel, cross-reactivity to JCV was also measured using stimulation with JCV-VLP, the JCV-VP1-peptide pool and a pool of HLA-B7-binding peptides. HLA-B7-binding peptides were considered for two reasons.

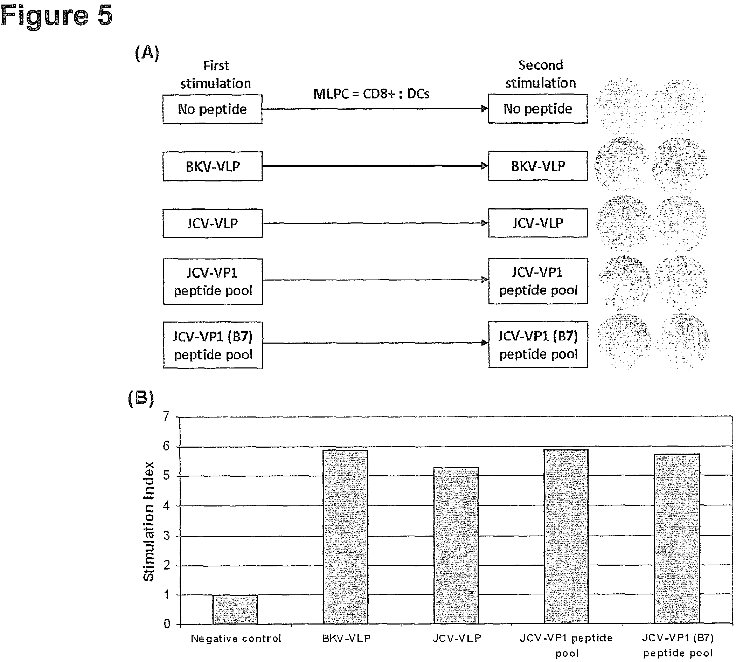

To this end, the amino acid sequence of the JCV-VP1-protein was screened and six nonamer HLA-B7-binding peptides were predicted on the basis of prediction scores using two programs SYFPEITHI (http://www.syfpeithi.de/) (score of more than 18) and BIMAS (http://www-bimas.cit.nih.gov/molbio/hla_bind/) (score of more than 180). In MLPCs, CD8⁺ T cells were stimulated using DCs loaded with JCV-VLP, the JCV-VP1-peptide pool and a pool of HLA-B7-binding peptides. In IFN-γ secretion assays we observed high T cell responses to both BKV-and JCV-VLP (Figure 5 A, B). Moreover, we observed equally high T cell responses to both the VP1-peptide pool and the pool of HLA-B7-binding peptides derived from JCV (Figure 5 A, B). A positive response to the pool of HLA-B7-binding peptides suggests the presence of possible T cell determinants in this pool.

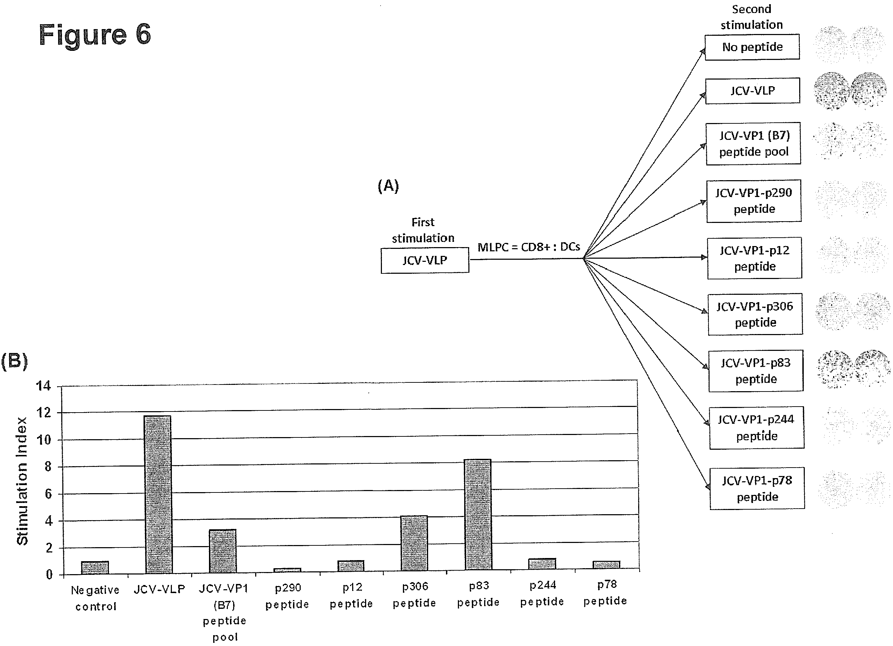

To identify further which one of the HLA-B7-binding peptides induced a positive CD8⁺ T cell response, MLPCs were performed using individual peptides. First, CD8⁺ T cells were stimulated using DCs loaded with JCV-VLP. Then, CD8⁺ T cells were restimulated in ELISPOT assays using JCV-VLP, the pool of HLA-B7-binding peptides and all the HLA-B7-binding peptides individually. In the ELISPOT assays, complete medium and SEB served as negative and positive controls respectively. We observed that there were significantly high T cell responses to JCV-VLP. Additionally, strong T cell responses were measured to the individual peptide p83 while moderate T cell responses were observed to p306-peptide (Figure 6 A, B). Other peptides showed rather low or no IFN-γ secretion. Since the first stimulation was effective through JCV-VLP loaded DCs, it was evident that all responding peptides were naturally processed.

We also observed T cell responses to the pool of HLA-B7-binding peptides. However, the T cell response was low when compared to the individual peptides and VLP (Figure 6 A, B). The lower response might be due to peptide competition (six peptides in a pool) for binding to the presenting HLA molecules.

In conclusion, in a patient with a previous history of HC we observed high T cell responses to both BKV-and JCV-VLP which indicates the possibility of cross-reactivity among BKV-and JCV-specific T cells. Furthermore, we found T cell specificities to the two predicted HLA-B7-binding peptides p83 and p306. Both peptides were demonstrated to be naturally processed.

### DISCUSSION

### - Peptide length

The effect of peptide length on the initiation of T cell responses is still under debate. In order to analyze whether HLA-transfectants could efficiently process longer peptides (15mer) and stimulate T cell responses, we used 9mer and 15mer peptides derived from two different viral antigens. It was observed that 15mer peptides can be efficiently processed and presented by both PBMCs and cell lines. This information was very crucial for establishing strategy for restriction analysis of antigenic peptides using HLA-transfectants (see Figure 3).

Using titrations of 9mer and 15mer peptides, we observed that 9mer peptides are superior in initiating T cell responses. At low concentrations, the dose-response curve of 15mer peptides was still in a linear phase while it was already in plateau with the same concentration of 9mer peptide. This indicates that for the initiation of T cell responses, higher concentrations of 15mer peptides is required when compared to 9mer peptides (data not shown).

### - Novel TCEs derived for JCV-VP1

In the present study, we explored CD8⁺ T cell responses with the objective to identify new TCEs specific for JCV toward vaccination approaches. For this purpose, we focused on two alleles, HLA-A2 and HLA-B7 expressed in 50% and in 25% of the Caucasian population, respectively. HLA-A2 and HLA-B7 positive HDs were first screened for the presence of T cell responses to JCV infection. Two routinely practiced methods for the measurement of JCV infection in individuals are anti-VLP antibodies (in the serum) and JCV-DNA in the urine (Jelcic et al., 2013). However, these two methods do not measure T cell responses. To select HDs based on the T cell reactivity to JCV, we therefore used the VP1-peptide pool as stimulus in a T cell assay (MLPC). VP1 is one out of five proteins of JCV, which has been considered to be highly immunogenic (Jelcic et al., 2013). Immunogenicity of VP1 was also confirmed in studies with BKV in HDs and patients. In this study, considering VP1-protein as the immunological target, we performed epitope mapping using an approach displayed in Figure 3.

### -JCV-VP1 overlapping peptides and the peptide pool

In a comprehensive epitope mapping strategy, we used JCV-VP1 overlapping peptides as matrix peptide sub-pools in ELISPOT assays (Figure 1). The use of overlapping peptides for T cell epitope mapping for other viruses has already been reported (see e.g. Walsh et al., 2013). In the experimental setting, we used 25,000 to 30,000 effector cells stimulated in MLPC and the IFN-γ secretion was characterized into weak, moderate and strong T cell responses on the basis of S.I. Based on this criteria, we detected JCV-VP1-specific CD8⁺ T cell responses in almost 73% (16 out of 22) of HDs.

### - Virus like particles (VLP)

To study cellular immune responses to JCV-VP1 and to map TCEs, we used VP1-VLP as primary stimulus. VLP are derived from VP1 and since VP1 constitutes almost 80% of the JCV-capsid, VLP shows morphological (Teunissen et al., 2013) and immunological resemblance to the virus in its natural form (Nicol et al., 2012). VLP can be safely used at the S1 level and using VLP allows the selection of naturally processed T cell specificities. While using peptides, one might find some specificity which is normally not presented by virus-infected cells, VLP on the other hand has to be first internalized and then processed by the proteasome machinery into peptides to bind to the HLA class I molecule. In this process, all the resulting T cell determinants presented by HLA class I molecules are not artificial but naturally processed.

### - Restriction analysis

Another important aspect in the identification of TCEs was to perform HLA restricted peptide recognition. Employing HLA-transfectants in ELISPOT assays was the key step because that way we were selecting a particular HLA restricted T cell specificity out of many VLP-stimulated HLA class I restricted T cell specificities.

### - Immune responses to peptides p36 and p100

Previously, two CD8⁺ TCEs (p36 and p100) have been described in HLA-A2 positive donors (Koralnik et al., 2002; Du Pasquier et al., 2003). Using the p100-peptide at different concentrations we characterized T cells with high-affinity TCR. In our experimental setting, the p100-peptide was represented by the 15mer peptide OP25 and matrix sub-pools 5 and 13 (Figure 1). The p36-peptide was covered by the 15mer peptide OP9 and matrix sub-pools 9 and 11. We found CD8⁺ T cells specific to both immunodominant peptides in more than one-third HLA-A2 positive donors (Table 1).

### - New TCEs

In addition to two known epitopes, we observed several other HLA-A2 and HLA-B7 restricted T cell specificities (Table 1 and Table 2). Some of them tested positive in more than 10% donors, e.g. the HLA-A2 restricted peptides OP28, OP29, OP72, OP78, p113 and the HLA-B7 restricted peptides OP23, OP53, p290, and p83. Out of these TCEs, some peptides tested positive in more than 25% of HDs (Table 3). Along with p36 and p100-peptides, these peptides can be also considered as immunodominant. Of most interest are the two neighbouring overlapping 15mer peptides OP28 and OP29 which contain the common 9mer sequence p113 (IGVTSLMNV). Using this 9mer peptide as stimulus we observed high frequencies of positive donors (25%) and strong T cell responses (Table 4).

### - Cross-reactivity and new TCEs using patients' PBMCs

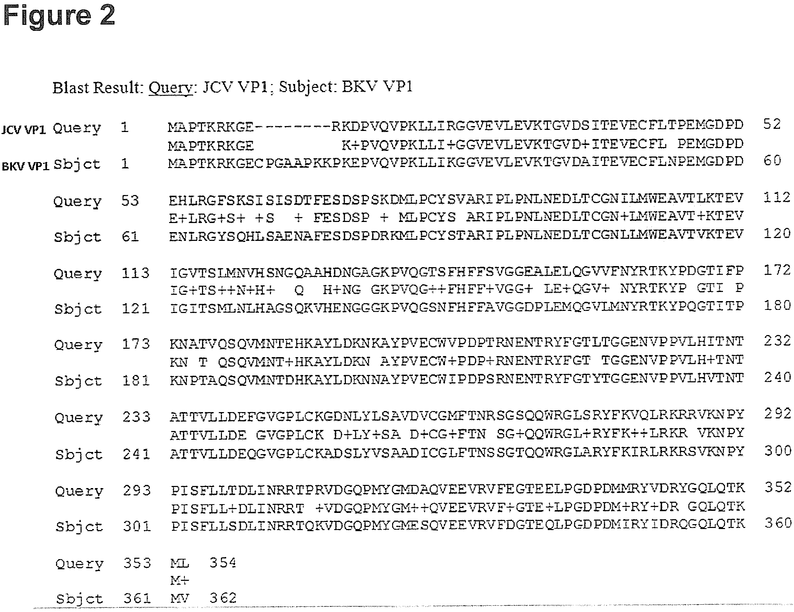

A patient, who has already experienced an active phase of infection, was used as a model to map TCEs, essentially due to the presence of high precursor frequencies of virus-specific T cells. We had the opportunity to use blood samples from an HC-affected patient for TCE mapping. Despite the fact that the patient had experienced BKV-induced HC and not PML (JCV-induced), our hypothesis to identify a TCE was based on the cross-reactivity of T cells due to high sequence homology between these two viruses (Figure 2). For this purpose, we used JCV and BKV-VLP. Interestingly, we observed a similar CD8⁺ T cell response to both types of VLPs. Additionally, similar responses were observed to a pool of six predicted HLA-B7-binding peptides also (Figure 5). In order to find immunogenic peptides present in the pool, individual peptides were used after being primarily stimulated with JCV-VLP. Two individual peptides (p83 and p306) were found positive (Figure 27). The 9mer peptide p83 was strongly positive in one HD also where it showed high T cell responses in three independent experiments (Table 2).

### - JCV-specific cellular immunotherapies

To date, single case of T cell adoptive transfer against JCV-infection has been reported (Balduzzi et al., 2011). *In vitro* activated JCV-specific CTLs were introduced in a patient with clinical status of PML who showed great improvement after adoptive T cell transfer. In this case report, T cells were activated using peptide pool of VP1 and LT-antigen with no enrichment and isolation of virus-specific T cells. Bulk T cells, however, might lead to GvHD in immunosuppressed patients due to the presence of non-specific T cells. In our study, we defined some CD8⁺ TCEs and since the stimulation was induced by VLP such TCEs are naturally processed. Most importantly, adoptive transfer with viral epitope-specific T cells would greatly minimize the risk of GvHD in patients.

### Conclusions

Identification and characterization of polyomavirus-specific T cell epitopes is a prerequisite for the development of both peptide based vaccines and antigen-specific T cell therapies. Adoptive T cell transfer has shown promising clinical results for some virus-related infections

(e.g. CMV, EBV, adenovirus etc.). To this end, we exploited a comprehensive strategy including mapping of T cell epitopes using overlapping peptides combined with peptide matrix pools and algorithm programs. It was demonstrated that two immunodominant HLA-A2 restricted T cell specificities for VP1p36 and p100 peptides interact with high avidity and preserve the functional integrity for weeks after *in vitro* culture. Novel, naturally processed T cell epitopes were identified and constitute candidates for future cellular immunotherapies. Our study focused on HLA-A2 and HLA-B7 alleles and as such covers the majority of the Caucasian population. Cross-reactivity, as demonstrated for BKV-and JCV-specific T cells, may also facilitate targeting both polyomaviruses-related diseases simultaneously.

The following examples and drawings illustrate the present invention without, however, limiting the same thereto.

### BRIEF DESCRIPTION OF THE DRAWINGS

**Figure 1****.** *Overlapping peptide sequences and matrix peptide sub-pools covering the JCV-VP1 sequence.*
   (A) General scheme of overlapping peptide sequences. Each peptide is 15 amino acid residues long and overlaps with the next peptide by 11 amino acid residues (bracket 1 and 2).
   (B) Instead of each peptide being tested individually, a total of 19 matrix peptide sub-pools were made from 86 JCV-VP1 overlapping peptides. Thus, each individual peptide is present in two different sub-pools. For example, when peptide sub-pools 5 and 13 are both positive (highlighted), all individual peptides present in these two peptide sub-pools could be antigenic, however the intersection of both stimulatory sub-pools indicates peptide number 25 (OP25) as antigenic peptide.
**Figure 2****.** *Alignment of JCV-VP1 versus BKV-VP1 using the data base protein BLAST.* Query and subject represents JCV-VP1 and BKV-VP1 amino acid sequences. Highlighted are the homologous amino acid sequences for JCV-VP1-p100 (ILMWEAVTL) and BKV-VP1-p108 (LLMWEAVTV) peptides respectively.
**Figure 3****.** *Systematic scheme of T cell epitope mapping.*
   (A) Identification of T cell specificities using the peptide matrix pool approach.
   (B) Determination of the restriction molecule using selected individual 15mers in combination with HLA-transfectants.
   (C) Determination of the fine epitope specificities using predicted 9mer peptides.
**Figure 4****.** *T cell epitope screening using matrix peptide sub-pools.*
   (A) ELISPOT wells showing negative and positive controls as well as wells stimulated by nineteen different peptide sub-pools in duplicate. Experimental wells stimulated with peptide sub-pools 10 and 11 were marked as positive results.
   (B) Histogram with bars representing IFN-γ secreting cells per 25,000 cells. Based on the T cell responses, two matrix peptide sub-pools were selected (red ellipses) and
   (C) the corresponding individual 15mer peptide common to both sub-pools was identified (OP 10) in the matrix grid.
**Figure 5****.** *T cell responses to JCV and BKV in a HLA-B7 positive patient.*
   IFN-γ ELISPOT assays were performed using autologous DCs loaded with complete medium, BKV-VLP, JCV-VLP, the JCV-VP1-peptide pool and a B7-peptide pool. IFN-γ secretion by T cells as original data in ELISPOT plate wells as well as histogram with bars indicating the respective S.I.
**Figure 6****.** *TCE mapping in a patient.*
   IFN-γ ELISPOT assays were performed using autologous DCs loaded with complete medium, JCV-VLP, the pool of HLA-B7-binding peptides and six individual HLA-B7-binding peptides. IFN-γ secretion by T cells as original data in ELISPOT plate wells as well as histogram with bars indicating the respective S.I.

### EXAMPLES

### 1. Materials and Methods

### 1.1 Isolation of PBMCs

Peripheral blood were diluted in PBS (Invitrogen, Darmstadt, Germany) and peripheral blood mononuclear cells (PBMCs) were separated using FicoLite-H separating solution (LINARIS, Dossenheim, Germany) by density gradient centrifugation. PBMCs were cryopreserved in 90% heat-inactivated fetal bovine serum (PAA, Linz, Austria) and 10% DMSO (Sigma-Aldrich, Steinheim, Germany) according to standard protocols.

### 1.2 Mixed lymphocyte peptide culture (MLPC)

Cryopreserved PBMCs were thawed, washed once with complete medium consists of Roswell Park Memorial Institute (RPMI-1640) containing 1% penicillin/streptomycin (Invitrogen Gibco, Grand Island, USA) and 1% L-Glutamine (Biochrom AG). Cells were then washed with separation buffer containing MACS buffer (AutoMACS rinsing solution, Miltenyi Biotec, Bergisch-Gladbach, Germany) and 0.5% bovine serum albumin (BSA, Miltenyi Biotec, Bergisch-Gladbach, Germany). CD8⁺ and CD8⁻ T cells were separated as positive and negative fraction through a MACS column (Miltenyi). CD8⁻ cells, which served as APCs were irradiated with 30 Gy and loaded with appropriate peptides, peptide pool or proteins. CD8⁻ cells cultured in complete medium without any peptide stimulations served as negative control. After 2 hours of peptide loading, CD8⁻ cells were co-cultured with CD8⁺ T cells in a ratio of 4:1 (20x10⁵: 5x10⁵) in 24-well plates. The ratio of 4:1 for CD8- and CD8⁺ T cells were also maintained when MLPC was done in 96-well plates. Plates were incubated overnight at 37°C, 5% CO₂ and supplemented with 2.5 ng/ml IL-2 (Sigma Aldrich, Steinheim, Germany) and 20 ng/mL IL-7 (Miltenyi Biotec, Bergisch-Gladbach, Germany). Cells were incubated further for 6 more days before harvesting for the experimental assays. For some assays, cells were restimulated with peptide loaded autologous PBMCs or K562 cells each week. Each well was fed with IL-2 and Il-7 every 2-3 days during this procedure.

### 1.3 IFN-γ ELISPOT assay

ELISPOT plates (Multiscreen IP 96-well plates, Millipore, Massachusetts, USA) were first pre-wet activated by adding 15 µl, 35% ethanol, washed with PBS and then coated with anti-human IFN-γ (Mabtech, Nacka Strand, Sweden) re-suspended in PBS. Plates were then incubated overnight at 4°C. Second day plates were washed with PBS to remove unbound antibodies and then blocked with PBS containing 10% FBS for 2 hours at RT. Autologous PBMCs and K562 cells loaded with peptides were used as target cells. Appropriate negative and positive control wells were prepared with complete medium and SEB-stimulation respectively. Effector cells from the cultured wells were then co-cultured with peptide loaded target cells in ELISPOT plate and incubated at 37°C, 5% CO₂ overnight. Third day plates were washed with PBS containing 0.05% tween 20 (Sigma-Aldrich) and biotin-linked secondary antibody was added in each well followed by 2 hours incubation at RT. Again after wash with PBS containing 0.05% tween 20, streptavidin-alkaline phosphatase (ALP) or streptavidin-horseradish peroxidase (HRP) was added and incubated for 2 hours at RT. Washes were first done with PBS containing 0.05% tween 20, followed by once with PBS. Substrate solution was prepared, added and reaction was developed for 3 minutes until visible spots were detected in positive control wells. Reaction was stopped with tap water and plate was air dried. Visible spots were counted and analyzed using an automated ELISPOT reader (CTL, Bonn, Germany).

The features disclosed in the foregoing description, in the claims and/or in the accompanying drawings may, both separately and in any combination thereof, be material for realizing the invention in diverse forms thereof.

### REFERENCES

Balduzzi, A., G. Lucchini, et al. (2011). "Polyomavirus JC-targeted T-cell therapy for progressive multiple leukoencephalopathy in a hematopoietic cell transplantation recipient." Bone Marrow Transplant 46(7): 987-992
Bienaime, A., P. Colson, et al. (2006). "Progressive multifocal leukoencephalopathy in HIV-2-infected patient." AIDS 20(9): 1342-1343.
Carson, K. R., D. Focosi, et al. (2009). "Monoclonal antibody-associated progressive multifocal leucoencephalopathy in patients treated with rituximab, natalizumab, and efalizumab: a Review from the Research on Adverse Drug Events and Reports (RADAR) Project." Lancet Oncol 10(8): 816-824.
Cettomai, D. and J. C. McArthur (2009). "Mirtazapine use in human immunodeficiency virus-infected patients with progressive multifocal leukoencephalopathy." Arch Neurol 66(2): 255-258.
Chesters, P. M., J. Heritage, et al. (1983). "Persistence of DNA sequences of BK virus and JC virus in normal human tissues and in diseased tissues." J Infect Dis 147(4): 676-684.
De Luca, A., A. Ammassari, et al. (2008). "Cidofovir in addition to antiretroviral treatment is not effective for AIDS-associated progressive multifocal leukoencephalopathy: a multicohort analysis." AIDS 22(14): 1759-1767.
Du Pasquier, R. A., M. J. Kuroda, et al. (2003). "Low frequency of cytotoxic T lymphocytes against the novel HLA-A*0201-restricted JC virus epitope VP1(p36) in patients with proven or possible progressive multifocal leukoencephalopathy." J Virol 77(22): 11918-11926.
Ferenczy, M. W., L. J. Marshall, et al. (2012). "Molecular biology, epidemiology, and pathogenesis of progressive multifocal leukoencephalopathy, the JC virus-induced demyelinating disease of the human brain." Clin Microbiol Rev 25(3): 471-506.
Focosi, D., R. E. Kast, et al. (2007). "Risperidone-induced reduction in JC viruria as a surrogate marker for efficacy against progressive multifocal leukoencephalopathy and hemorrhagic cystitis." J Clin Virol 39(1): 63-64.
Garcia-Suarez, J., D. de Miguel, et al. (2005). "Changes in the natural history of progressive multifocal leukoencephalopathy in HIV-negative lymphoproliferative disorders: impact of novel therapies." Am J Hematol 80(4): 271-281.
Gheuens, S., G. Pierone, et al. (2010). "Progressive multifocal leukoencephalopathy in individuals with minimal or occult immunosuppression." J Neurol Neurosurg Psychiatry 81 (3): 247-254.
Jelcic, I., L. Aly, et al. (2013). "T cell epitope mapping of JC polyoma virus-encoded proteome reveals reduced T cell responses in HLA-DRB1*04:01+ donors." J Virol 87(6): 3393-3408.
Kharfan-Dabaja, M. A., E. Ayala, et al. (2007). "Two cases of progressive multifocal leukoencephalopathy after allogeneic hematopoietic cell transplantation and a review of the literature." Bone Marrow Transplant 39(2): 101-107.
Kleinschmidt-DeMasters, B. K. and K. L. Tyler (2005). "Progressive multifocal leukoencephalopathy complicating treatment with natalizumab and interferon beta-1a for multiple sclerosis." N Engl J Med 353(4): 369-374.
Koralnik, I. J., R. A. Du Pasquier, et al. (2002). "Association of prolonged survival in HLA-A2+ progressive multifocal leukoencephalopathy patients with a CTL response specific for a commonly recognized JC virus epitope." J Immunol 168(1): 499-504.
Kunschner, L. and T. F. Scott (2005). "Sustained recovery of progressive multifocal leukoencephalopathy after treatment with IL-2." Neurology 65(9): 1510.
Langer-Gould, A., S. W. Atlas, et al. (2005). "Progressive multifocal leukoencephalopathy in a patient treated with natalizumab." N Engl J Med 353(4): 375-381.
Lewden, C., T. May, et al. (2008). "Changes in causes of death among adults infected by HIV between 2000 and 2005: The "Mortalite 2000 and 2005" surveys (ANRS EN19 and Mortavic)." J Acquir Immune Defic Syndr 48(5): 590-598.
Nicol, J. T., A. Touze, et al. (2012). "Seroprevalence and cross-reactivity of human polyomavirus 9." Emerg Infect Dis 18(8): 1329-1332.
Padgett, B. L., D. L. Walker, et al. (1971). "Cultivation of papova-like virus from human brain with progressive multifocal leucoencephalopathy." Lancet 1(7712): 1257-1260.
Shitrit, D., N. Lev, et al. (2005). "Progressive multifocal leukoencephalopathy in transplant recipients." Transpl Int 17(11): 658-665.
Stoner, G. L., H. T. Agostini, et al. (1998). "Detection of JC virus in two African cases of progressive multifocal leukoencephalopathy including identification of JCV type 3 in a Gambian AIDS patient." J Med Microbiol 47(8): 733-742.
Sweet, B. H. and M. R. Hilleman (1960). "The vacuolating virus, S.V. 40." Proc Soc Exp Biol Med 105: 420-427.
Tavazzi, E., M. K. White, et al. (2012). "Progressive multifocal leukoencephalopathy: clinical and molecular aspects." Rev Med Virol 22(1): 18-32.
Teunissen, E. A., M. de Raad, et al. (2013). "Production and biomedical applications of virus-like particles derived from polyomaviruses." J Control Release 172(1): 305-321.
Walsh, K. B., J. Sidney, et al. (2013). "CD8+ T-cell epitope mapping for pneumonia virus of mice in H-2b mice." J Virol 87(17): 9949-9952.

## Claims

1. A vaccine comprising at least one compound,
said at least one compound comprising at least one antigenic determinant or epitope of polyomavirus capsid protein (VP1),
wherein said at least one VP1 antigenic determinant or epitope is selected from
| | |
|---|---|
| IGVTSLMNV | SEQ ID NO. 1, |
| IGITSMLNL | SEQ ID NO. 2, |
| IGVTSLMNVHSNGQA | SEQ ID NO. 3, |
| IGITSMLNLHAGSQK | SEQ ID NO. 4, |
| KTEVIGVTSLMNVHS | SEQ ID NO. 5, |
| KTEVIGITSMLNLHA | SEQ ID NO. 6, |
| KRRVKNPYPISFLLT | SEQ ID NO. 7, |
| KRSVKNPYPISFLLS | SEQ ID NO. 8, |
| KNPYPISFLLTDLIN | SEQ ID NO. 9, |
| KNPYPISFLLSDLIN | SEQ ID NO. 10, |
| VARIPLPNL | SEQ ID NO. 11, |
| TARIPLPNL | SEQ ID NO. 12, and |
| KNPYPISFL | SEQ ID NO. 13. |

2. The vaccine according to claim 1, wherein the antigenic determinant or epitope is a CD8+ T cell epitope,
preferably HLA-A2-restricted or HLA-B7-restricted.

3. The vaccine according to claim 1 or 2, wherein the amino acid sequence of the antigenic determinant or epitope has a length of at least 9 amino acids,
wherein the amino acid sequence of the antigenic determinant or epitope has a length of 9 to 100 amino acids, preferably 9 to 50 amino acids, more preferably 9 to 25 amino acids, such as 15 amino acids.

4. The vaccine according to any one of claims 1 to 3, wherein said at least one compound is a peptide, preferably consisting of said at least one VP1 antigenic determinant or epitope.

5. The vaccine according to claim 4, wherein the vaccine comprises further component(s), such as tag(s), linker, label(s), N- and/or C-terminal modification(s), drug(s), agent(s), and/or combinations thereof,
and/or wherein the amino acid sequence comprises modified amino acid(s), unnatural amino acid(s) or peptidomimetic(s).

6. The vaccine according to any one of claims 1 to 5, comprising at least two compounds, such as at least two peptides, as defined in any of claims 1 to 5.

7. The vaccine according to any one of claims 1 to 6, further comprising carrier(s), such as a virus like particle (VLP), and/or adjuvant(s).

8. A nucleic acid molecule coding for at least one peptide as defined in any one of the claims 1 to 5 or a plasmid comprising at least one such nucleic acid molecule.

9. The nucleic acid of claim 8 for use as a vaccine,
and/or the vaccine according to any one of claims 1 to 7 or the nucleic acid of claim 8 for use for the prevention and/or treatment of an infection or reactivation with polyoma virus(es), wherein the polyoma virus is preferably JC virus (JCV), BK virus (BKV).

10. A compound comprising at least one antigenic determinant or epitope of polyomavirus capsid protein (VP1),
wherein said at least one VP1 antigenic determinant or epitope is selected from
| | |
|---|---|
| IGVTSLMNV | SEQ ID NO. 1, |
| IGITSMLNL | SEQ ID NO. 2, |
| IGVTSLMNVHSNGQA | SEQ ID NO. 3, |
| IGITSMLNLHAGSQK | SEQ ID NO. 4, |
| KTEVIGVTSLMNVHS | SEQ ID NO. 5, |
| KTEVIGITSMLNLHA | SEQ ID NO. 6, |
| KRRVKNPYPISFLLT | SEQ ID NO. 7, |
| KRSVKNPYPISFLLS | SEQ ID NO. 8, |
| KNPYPISFLLTDLIN | SEQ ID NO. 9, |
| KNPYPISFLLSDLIN | SEQ ID NO. 10, |
| VARIPLPNL | SEQ ID NO. 11, |
| TARIPLPNL | SEQ ID NO. 12, and |
| KNPYPISFL | SEQ ID NO. 13, |
for use for the prevention and/or treatment of an infection or reactivation with polyoma virus(es),
wherein the compound is preferably as defined in any one of claims 1 to 6,
wherein the polyoma virus is preferably JC virus (JCV), BK virus (BKV).

11. The vaccine according to any of claims 1 to 7 or 9, the nucleic acid according to claim 8 or 9 or the compound according to claim 10,
comprising vaccination, adoptive immunotherapy, T-cell receptor cloning and transfection and/or construction of chimeric antigen receptors.

12. The vaccine, the nucleic acid or the compound according to any one of claims 9 to 11, wherein the use is carried out in case of/during immunosuppression,
such as in patients after transplantation (such as stem cell or organ transplantation), HIV infection, in patients with solid tumors or hematological malignancies, and patients with rheumatologic disorders (preferably systemic lupus erythematosus (SLE)), lympho-proliferative disorders, during monoclonal antibody (mAb) therapy.

13. A method for the prevention and/or treatment of an infection or reactivation with polyoma viruses,
comprising the step of
administering a vaccine according to any one of claims 1 to 8, a nucleic acid of claim 9 or a compound as defined in claim 11 to a subject in need thereof,
wherein the polyoma virus is preferably JC virus (JCV), BK virus (BKV),
said method preferably comprising the administration
(1) via injection,
(2) pulsing of compound(s) of the invention (e.g. peptides) on dendritic cells (DCs) which are thereafter administered,
(3) stimulation of T cells with compound(s) of the invention (e.g. peptide) ex vivo and transfusion of such stimulated and potentially selected T cells,
(4) transfection of nucleic acid(s) of the invention (e.g. DNA or RNA encoding the peptide(s)) into antigen-presenting cells, such as DCs,
(5) cloning of T cells recognizing specifically the compound(s) of the invention (e.g. peptides), expansion of these T clones *ex vivo* followed by injection,
(6) molecular cloning of a T cell receptor (TCR) from the T cell clones of (5) and transfection of such TCRs into autochthonous T lymphocytes of the subject in need thereof / the patient,
wherein the subject in need thereof is preferably an immunosuppressive patient,
such as a patient after transplantation (such as stem cell or organ transplantation, a HIV-infected patient, a patient with solid tumors or hematological malignancies or a patient with rheumatologic disorders (such as SLE), lympho-proliferative disorders, during monoclonal antibody (mAb) therapy.

14. The method of claim 13, comprising vaccination, adoptive immunotherapy, T-cell receptor cloning and transfection and/or construction of chimeric antigen receptors.

15. A compound comprising at least one antigenic determinant or epitope of polyomavirus capsid protein (VP1),
wherein said at least one VP 1 antigenic determinant or epitope is selected from
| | |
|---|---|
| IGVTSLMNV | SEQ ID NO. 1, |
| IGITSMLNL | SEQ ID NO. 2, |
| IGVTSLMNVHSNGQA | SEQ ID NO. 3, |
| IGITSMLNLHAGSQK | SEQ ID NO. 4, |
| KTEVIGVTSLMNVHS | SEQ ID NO. 5, |
| KTEVIGITSMLNLHA | SEQ ID NO. 6, |
| KRRVKNPYPISFLLT | SEQ ID NO. 7, |
| KRSVKNPYPISFLLS | SEQ ID NO. 8, |
| KNPYPISFLLTDLIN | SEQ ID NO. 9, |
| KNPYPISFLLSDLIN | SEQ ID NO. 10, |
| VARIPLPNL | SEQ ID NO. 11, and |
| TARIPLPNL | SEQ ID NO. 12, |
wherein the antigenic determinant or epitope is preferably a CD8+ T cell epitope,
more preferably HLA-A2-restricted or HLA-B7-restricted.

16. The compound according to claim 15, wherein the amino acid sequence of the antigenic determinant or epitope has a length of at least 9 amino acids,
wherein the amino acid sequence of the antigenic determinant or epitope has a length of 9 to 100 amino acids, preferably 9 to 50 amino acids, more preferably 9 to 25 amino acids, such as 15 amino acids,
wherein the compound is preferably a peptide, more preferably consisting of said at least one VP1 antigenic determinant or epitope,
and/or wherein the compound preferably comprises further component(s), such as tag(s), linker, label(s), N- and/or C-terminal modification(s), drug(s), agent(s), and/or combinations thereof,
and/or wherein the amino acid sequence preferably comprises modified amino acid(s), unnatural amino acid(s) or peptidomimetic(s).


SEQUENCE LISTING

<110>  Ruprecht-Karls-Universität Heidelberg

<120>  CD8+ T-cell epitopes from polyomavirus capsid protein VP1 for
       vaccination

<130>  U30549EP

<160>  64

<170>  PatentIn version 3.5

<210>  1
<211>  9
<212>  PRT
<213>  JC virus

<400>  1

Ile Gly Val Thr Ser Leu Met Asn Val
1               5

<210>  2
<211>  9
<212>  PRT
<213>  BK virus

<400>  2

Ile Gly Ile Thr Ser Met Leu Asn Leu
1               5

<210>  3
<211>  15
<212>  PRT
<213>  JC virus

<400>  3

Ile Gly Val Thr Ser Leu Met Asn Val His Ser Asn Gly Gln Ala
1               5                   10                  15

<210>  4
<211>  15
<212>  PRT
<213>  BK virus

<400>  4

Ile Gly Ile Thr Ser Met Leu Asn Leu His Ala Gly Ser Gln Lys
1               5                   10                  15

<210>  5
<211>  15
<212>  PRT
<213>  JC virus

<400>  5

Lys Thr Glu Val Ile Gly Val Thr Ser Leu Met Asn Val His Ser
1               5                   10                  15

<210>  6
<211>  15
<212>  PRT
<213>  BK virus

<400>  6

Lys Thr Glu Val Ile Gly Ile Thr Ser Met Leu Asn Leu His Ala
1               5                   10                  15

<210>  7
<211>  15
<212>  PRT
<213>  JC virus

<400>  7

Lys Arg Arg Val Lys Asn Pro Tyr Pro Ile Ser Phe Leu Leu Thr
1               5                   10                  15

<210>  8
<211>  15
<212>  PRT
<213>  BK virus

<400>  8

Lys Arg Ser Val Lys Asn Pro Tyr Pro Ile Ser Phe Leu Leu Ser
1               5                   10                  15

<210>  9
<211>  15
<212>  PRT
<213>  JC virus

<400>  9

Lys Asn Pro Tyr Pro Ile Ser Phe Leu Leu Thr Asp Leu Ile Asn
1               5                   10                  15

<210>  10
<211>  15
<212>  PRT
<213>  BK virus

<400>  10

Lys Asn Pro Tyr Pro Ile Ser Phe Leu Leu Ser Asp Leu Ile Asn
1               5                   10                  15

<210>  11
<211>  9
<212>  PRT

<213>  JC virus

<400>  11

Val Ala Arg Ile Pro Leu Pro Asn Leu
1               5

<210>  12
<211>  9
<212>  PRT
<213>  BK virus

<400>  12

Thr Ala Arg Ile Pro Leu Pro Asn Leu
1               5

<210>  13
<211>  9
<212>  PRT
<213>  JC virus

<400>  13

Lys Asn Pro Tyr Pro Ile Ser Phe Leu
1               5

<210>  14
<211>  354
<212>  PRT
<213>  JC virus

<400>  14

Met Ala Pro Thr Lys Arg Lys Gly Glu Arg Lys Asp Pro Val Gln Val
1               5                   10                  15

Pro Lys Leu Leu Ile Arg Gly Gly Val Glu Val Leu Glu Val Lys Thr
            20                  25                  30

Gly Val Asp Ser Ile Thr Glu Val Glu Cys Phe Leu Thr Pro Glu Met
        35                  40                  45

Gly Asp Pro Asp Glu His Leu Arg Gly Phe Ser Lys Ser Ile Ser Ile
    50                  55                  60

Ser Asp Thr Phe Glu Ser Asp Ser Pro Asn Arg Asp Met Leu Pro Cys
65                  70                  75                  80

Tyr Ser Val Ala Arg Ile Pro Leu Pro Asn Leu Asn Glu Asp Leu Thr
                85                  90                  95

Cys Gly Asn Ile Leu Met Trp Glu Ala Val Thr Leu Lys Thr Glu Val
            100                 105                 110

Ile Gly Val Thr Ser Leu Met Asn Val His Ser Asn Gly Gln Ala Thr
        115                 120                 125

His Asp Asn Gly Ala Gly Lys Pro Val Gln Gly Thr Ser Phe His Phe
    130                 135                 140

Phe Ser Val Gly Gly Glu Ala Leu Glu Leu Gln Gly Val Leu Phe Asn
145                 150                 155                 160

Tyr Arg Thr Lys Tyr Pro Asp Gly Thr Ile Phe Pro Lys Asn Ala Thr
                165                 170                 175

Val Gln Ser Gln Val Met Asn Thr Glu His Lys Ala Tyr Leu Asp Lys
            180                 185                 190

Asn Lys Ala Tyr Pro Val Glu Cys Trp Val Pro Asp Pro Thr Arg Asn
        195                 200                 205

Glu Asn Thr Arg Tyr Phe Gly Thr Leu Thr Gly Gly Glu Asn Val Pro
    210                 215                 220

Pro Val Leu His Ile Thr Asn Thr Ala Thr Thr Val Leu Leu Asp Glu
225                 230                 235                 240

Phe Gly Val Gly Pro Leu Cys Lys Gly Asp Asn Leu Tyr Leu Ser Ala
                245                 250                 255

Val Asp Val Cys Gly Met Phe Thr Asn Arg Ser Gly Ser Gln Gln Trp
            260                 265                 270

Arg Gly Leu Ser Arg Tyr Phe Lys Val Gln Leu Arg Lys Arg Arg Val
        275                 280                 285

Lys Asn Pro Tyr Pro Ile Ser Phe Leu Leu Thr Asp Leu Ile Asn Arg
    290                 295                 300

Arg Thr Pro Arg Val Asp Gly Gln Pro Met Tyr Gly Met Asp Ala Gln
305                 310                 315                 320

Val Glu Glu Val Arg Val Phe Glu Gly Thr Glu Glu Leu Pro Gly Asp
                325                 330                 335

Pro Asp Met Met Arg Tyr Val Asp Lys Tyr Gly Gln Leu Gln Thr Lys
            340                 345                 350

Met Leu

<210>  15
<211>  362
<212>  PRT
<213>  BK virus

<400>  15

Met Ala Pro Thr Lys Arg Lys Gly Glu Cys Pro Gly Ala Ala Pro Lys
1               5                   10                  15

Lys Pro Lys Glu Pro Val Gln Val Pro Lys Leu Leu Ile Lys Gly Gly
            20                  25                  30

Val Glu Val Leu Glu Val Lys Thr Gly Val Asp Ala Ile Thr Glu Val
        35                  40                  45

Glu Cys Phe Leu Asn Pro Glu Met Gly Asp Pro Asp Asp Asn Leu Arg
    50                  55                  60

Gly Tyr Ser Gln His Leu Ser Ala Glu Asn Ala Phe Glu Ser Asp Ser
65                  70                  75                  80

Pro Asp Arg Lys Met Leu Pro Cys Tyr Ser Thr Ala Arg Ile Pro Leu
                85                  90                  95

Pro Asn Leu Asn Glu Asp Leu Thr Cys Gly Asn Leu Leu Met Trp Glu
            100                 105                 110

Ala Val Thr Val Lys Thr Glu Val Ile Gly Ile Thr Ser Met Leu Asn
        115                 120                 125

Leu His Ala Gly Ser Gln Lys Val His Glu Asn Gly Gly Gly Lys Pro
    130                 135                 140

Val Gln Gly Ser Asn Phe His Phe Phe Ala Val Gly Gly Asp Pro Leu
145                 150                 155                 160

Glu Met Gln Gly Val Leu Met Asn Tyr Arg Thr Lys Tyr Pro Gln Gly
                165                 170                 175

Thr Ile Thr Pro Lys Asn Pro Thr Ala Gln Ser Gln Val Met Asn Thr
            180                 185                 190

Asp His Lys Ala Tyr Leu Asp Lys Asn Asn Ala Tyr Pro Val Glu Cys
        195                 200                 205

Trp Ile Pro Asp Pro Ser Arg Asn Glu Asn Thr Arg Tyr Phe Gly Thr

210                 215                 220

Tyr Thr Gly Gly Glu Asn Val Pro Pro Val Leu His Val Thr Asn Thr
225                 230                 235                 240

Ala Thr Thr Val Leu Leu Asp Glu Gln Gly Val Gly Pro Leu Cys Lys
                245                 250                 255

Ala Asp Ser Leu Tyr Val Ser Ala Ala Asp Ile Cys Gly Leu Phe Thr
            260                 265                 270

Asn Ser Ser Gly Thr Gln Gln Trp Arg Gly Leu Ala Arg Tyr Phe Lys
        275                 280                 285

Ile Arg Leu Arg Lys Arg Ser Val Lys Asn Pro Tyr Pro Ile Ser Phe
    290                 295                 300

Leu Leu Ser Asp Leu Ile Asn Arg Arg Thr Gln Lys Val Asp Gly Gln
305                 310                 315                 320

Pro Met Tyr Gly Met Glu Ser Gln Val Glu Glu Val Arg Val Phe Asp
                325                 330                 335

Gly Thr Glu Gln Leu Pro Gly Asp Pro Asp Met Ile Arg Tyr Ile Asp
            340                 345                 350

Arg Gln Gly Gln Leu Gln Thr Lys Met Val
        355                 360

<210>  16
<211>  15
<212>  PRT
<213>  JC virus

<400>  16

Gly Val Asp Ser Ile Thr Glu Val Glu Cys Phe Leu Thr Pro Glu
1               5                   10                  15

<210>  17
<211>  15
<212>  PRT
<213>  JC virus

<400>  17

Met Leu Pro Cys Tyr Ser Val Ala Arg Ile Pro Leu Pro Asn Leu
1               5                   10                  15

<210>  18
<211>  15

<212>  PRT
<213>  JC virus

<400>  18

Cys Gly Asn Ile Leu Met Trp Glu Ala Val Thr Leu Lys Thr Glu
1               5                   10                  15


<210>  19
<211>  15
<212>  PRT
<213>  JC virus

<400>  19

Pro Val Glu Cys Trp Val Pro Asp Pro Thr Arg Asn Glu Asn Thr
1               5                   10                  15


<210>  20
<211>  15
<212>  PRT
<213>  JC virus

<400>  20

Pro Thr Arg Asn Glu Asn Thr Arg Tyr Phe Gly Thr Leu Thr Gly
1               5                   10                  15


<210>  21
<211>  15
<212>  PRT
<213>  JC virus

<400>  21

Val Asp Val Cys Gly Met Phe Thr Asn Arg Ser Gly Ser Gln Gln
1               5                   10                  15


<210>  22
<211>  15
<212>  PRT
<213>  JC virus

<400>  22

Lys Asn Pro Tyr Pro Ile Ser Phe Leu Leu Thr Asp Leu Ile Asn
1               5                   10                  15


<210>  23
<211>  15
<212>  PRT
<213>  JC virus

<400>  23

Val Asp Gly Gln Pro Met Tyr Gly Met Asp Ala Gln Val Glu Glu
1               5                   10                  15

<210>  24
<211>  15
<212>  PRT
<213>  JC virus

<400>  24

Pro Met Tyr Gly Met Asp Ala Gln Val Glu Glu Val Arg Val Phe
1               5                   10                  15


<210>  25
<211>  15
<212>  PRT
<213>  JC virus

<400>  25

Gly Thr Glu Glu Leu Pro Gly Asp Pro Asp Met Met Arg Tyr Val
1               5                   10                  15


<210>  26
<211>  10
<212>  PRT
<213>  JC virus

<400>  26

Ile Leu Met Glu Trp Glu Ala Val Thr Leu
1               5                   10


<210>  27
<211>  9
<212>  PRT
<213>  JC virus

<400>  27

Ser Ile Thr Glu Val Glu Cys Phe Leu
1               5


<210>  28
<211>  9
<212>  PRT
<213>  JC virus

<400>  28

Ser Ile Thr Glu Val Glu Cys Phe Leu
1               5


<210>  29
<211>  9
<212>  PRT
<213>  JC virus

<400>  29

Val Ala Arg Ile Pro Leu Pro Asn Leu
1               5

<210>  30
<211>  9
<212>  PRT
<213>  JC virus

<400>  30

Ile Leu Met Trp Glu Ala Val Thr Leu
1               5

<210>  31
<211>  9
<212>  PRT
<213>  JC virus

<400>  31

Thr Glu Val Ile Gly Val Thr Ser Leu
1               5

<210>  32
<211>  9
<212>  PRT
<213>  JC virus

<400>  32

Trp Val Pro Asp Pro Thr Arg Asn Glu
1               5

<210>  33
<211>  9
<212>  PRT
<213>  JC virus

<400>  33

Glu Asn Thr Arg Tyr Phe Gly Thr Leu
1               5

<210>  34
<211>  9
<212>  PRT
<213>  JC virus

<400>  34

Gly Met Phe Thr Asn Arg Ser Gly Ser
1               5

<210>  35
<211>  9
<212>  PRT
<213>  JC virus

<400>  35

Pro Ile Ser Phe Leu Leu Thr Asp Leu
1               5


<210>  36
<211>  9
<212>  PRT
<213>  JC virus

<400>  36

Pro Met Tyr Gly Met Asp Ala Gln Val
1               5


<210>  37
<211>  9
<212>  PRT
<213>  JC virus

<400>  37

Gly Met Asp Ala Gln Val Glu Glu Val
1               5


<210>  38
<211>  9
<212>  PRT
<213>  JC virus

<400>  38

Glu Leu Pro Gly Asp Pro Asp Met Met
1               5


<210>  39
<211>  9
<212>  PRT
<213>  JC virus

<400>  39

Ile Gly Val Thr Ser Leu Met Asn Val
1               5


<210>  40
<211>  15
<212>  PRT
<213>  JC virus

<400>  40

Thr Pro Glu Met Gly Asp Pro Asp Glu His Leu Arg Gly Phe Ser
1               5                   10                  15


<210>  41

<211>  15
<212>  PRT
<213>  JC virus

<400>  41

Gly Asp Pro Asp Glu His Leu Arg Gly Phe Ser Lys Ser Ile Ser
1               5                   10                  15


<210>  42
<211>  15
<212>  PRT
<213>  JC virus

<400>  42

Glu Ser Asp Ser Pro Asn Arg Asp Met Leu Pro Cys Tyr Ser Val
1               5                   10                  15


<210>  43
<211>  15
<212>  PRT
<213>  JC virus

<400>  43

Pro Asn Leu Asn Glu Asp Leu Thr Cys Gly Asn Ile Leu Met Trp
1               5                   10                  15


<210>  44
<211>  15
<212>  PRT
<213>  JC virus

<400>  44

His Asp Asn Gly Ala Gly Lys Pro Val Gln Gly Thr Ser Phe His
1               5                   10                  15


<210>  45
<211>  15
<212>  PRT
<213>  JC virus

<400>  45

Val Leu Phe Asn Tyr Arg Thr Lys Tyr Pro Asp Gly Thr Ile Phe
1               5                   10                  15


<210>  46
<211>  15
<212>  PRT
<213>  JC virus

<400>  46

Glu Asn Thr Arg Tyr Phe Gly Thr Leu Thr Gly Gly Glu Asn Val
1               5                   10                  15

<210>  47
<211>  15
<212>  PRT
<213>  JC virus

<400>  47

Pro Val Leu His Ile Thr Asn Thr Ala Thr Thr Val Leu Leu Asp
1               5                   10                  15


<210>  48
<211>  15
<212>  PRT
<213>  JC virus

<400>  48

Pro Leu Cys Lys Gly Asp Asn Leu Tyr Leu Ser Ala Val Asp Val
1               5                   10                  15


<210>  49
<211>  15
<212>  PRT
<213>  JC virus

<400>  49

Leu Ile Asn Arg Arg Thr Pro Arg Val Asp Gly Gln Pro Met Tyr
1               5                   10                  15


<210>  50
<211>  15
<212>  PRT
<213>  JC virus

<400>  50

Arg Val Phe Glu Gly Thr Glu Glu Leu Pro Gly Asp Pro Asp Met
1               5                   10                  15


<210>  51
<211>  9
<212>  PRT
<213>  JC virus

<400>  51

Gly Pro Leu Cys Lys Gly Asp Asn Leu
1               5


<210>  52
<211>  9
<212>  PRT
<213>  JC virus

<400>  52

Asn Pro Tyr Pro Ile Ser Phe Leu Leu
1               5

<210>  53
<211>  9
<212>  PRT
<213>  JC virus

<400>  53

Asp Pro Asp Glu His Leu Arg Gly Phe
1               5

<210>  54
<211>  9
<212>  PRT
<213>  JC virus

<400>  54

Asp Pro Asp Glu His Leu Arg Gly Phe
1               5

<210>  55
<211>  9
<212>  PRT
<213>  JC virus

<400>  55

Ser Asp Ser Pro Asn Arg Asp Met Leu
1               5

<210>  56
<211>  9
<212>  PRT
<213>  JC virus

<400>  56

Glu Asp Leu Thr Cys Gly Asn Ile Leu
1               5

<210>  57
<211>  9
<212>  PRT
<213>  JC virus

<400>  57

Lys Pro Val Gln Gly Thr Ser Phe His
1               5

<210>  58
<211>  9
<212>  PRT

<213>  JC virus

<400>  58

Arg Thr Lys Tyr Pro Asp Gly Thr Ile
1               5


<210>  59
<211>  9
<212>  PRT
<213>  JC virus

<400>  59

Glu Asn Thr Arg Tyr Phe Gly Thr Leu
1               5


<210>  60
<211>  9
<212>  PRT
<213>  JC virus

<400>  60

Ile Thr Asn Thr Ala Thr Thr Val Leu
1               5


<210>  61
<211>  9
<212>  PRT
<213>  JC virus

<400>  61

Leu Cys Lys Gly Asp Asn Leu Tyr Leu
1               5


<210>  62
<211>  9
<212>  PRT
<213>  JC virus

<400>  62

Thr Pro Arg Val Asp Gly Gln Pro Met
1               5


<210>  63
<211>  9
<212>  PRT
<213>  JC virus

<400>  63

Arg Val Phe Glu Gly Thr Glu Glu Leu
1               5

<210> 64
<211> 9
<212> PRT
<213> JC virus

<400> 64

Gly Pro Leu Cys Lys Gly Asp Asn Leu
1               5